# EUROPEAN PATENT SPECIFICATION

(11) **EP 0 550 645 B1**
(45) Date of publication and mention of the grant of the patent: **22.08.2001**
(21) Application number: 91918234.5
(22) Date of filing: 27.09.1991
(51) Int. Cl.: C12N 15/62, C12N 15/64, C12N 15/70, C07K 16/00, C07K 14/705, C12Q 1/70

(54) **SURFACE EXPRESSION LIBRARIES OF HETEROMERIC RECEPTORS**
OBERFLÄCHEN LOKALISIERTE EXPRESSIONSBANKEN VON HETEROMEREN REZEPTOREN
BANQUES DE RECEPTEURS HETEROMERES A EXPRESSION EN SURFACE

(30) Priority: 28.09.1990 US 590219
(43) Date of publication of application: 14.07.1993
(73) Proprietor: IXSYS, INC., San Diego, CA 92121 (US)
(72) Inventor: HUSE, William, D., Del Mar, CA 92014 (US)
(74) Representative: Goldin, Douglas Michael
(86) International application number: US9107149
(87) International publication number: WO9206204

(56) References cited:
- WO-A-88/06630
- WO-A-88/09344
- WO-A-89/01526
- WO-A-90/14424
- WO-A-90/14430
- WO-A-90/14443
- PROCEEDINGS OF THE NATIONAL ACADEMY OF SCIENCES OF USA. vol. 86, no. 10, 15 May 1989, WASHINGTON US pages 3833 - 3837 Orlandi, R. et al.; 'Cloning Immunoglobulin variable domains for expression by the polymerase chain reaction.'
- BIOCHEMICAL AND BIOPHYSICAL RESEARCH COMMUNICATIONS. vol. 160, no. 3, 15 May 1989, DULUTH, MINNESOTA US pages 1250 - 1256 Larrick, J.W. et al.; 'Rapid cloning of rearranged Immunoglobulin genes from human hybridoma cells using mixed primers and the ploymerase chain reaction.'
- Nucleic Acids Research, Vol. 12, No. 9, issued September 1984, BOSS et al., "Assembly of functional antibodies from immunoglobulin heavy and light chains synthesized in E. coli", pages 3791-3806, see the Abstract.
- Proceedings of the National Academy of Sciences, Vol. 86, issued August 1989, SASTRY et al., "Cloning of the immunological repertoire in Escherichia coli for generation of monoclonal catalytic antibodies: Construction of a heavy chain variable-region specific cDNA library", pages 5728-5732, see the Abstract.
- Science, Vol. 246, issued 08 December 1989, HUSE et al., "Generation of a Large Combinatorial Library of the Immunoglobulin Repertoire in Phage Lambda", pages 1275-1281, see entire document.
- Gene, Vol. 73, issued 1988, PARMLEY et al., "Antibody-selectable filamentous fd phage vectors: affinity purification of target genes", pages 305-318, see entire document.

### SEQUENCE LISTING

(1) GENERAL INFORMATION:
   (i) APPLICANT: HUSE, WILLIAM D.
   (ii) TITLE OF INVENTION: SURFACE EXPRESSION LIBRARIES OF HETEROMERIC RECEPTORS
   (iii) NUMBER OF SEQUENCES: 75
   (iv) CORRESPONDENCE ADDRESS:
      (A) ADDRESSEE: PRETTY, SCHROEDER, BRUEGGEMANN & CLARK
      (B) STREET: 444 SO. FLOWER STREET, SUITE 200
      (C) CITY: LOS ANGELES
      (D) STATE: CALIFORNIA
      (E) COUNTRY: UNITED STATES
      (F) ZIP: 90071
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: CAMPBELL, CATHRYN A.
      (B) REGISTRATION NUMBER: 31,815
      (C) REFERENCE/DOCKET NUMBER: P31 8882
   (ix) TELECOMMUNICATION INFORMATION:
      (A) TELEPHONE: 619-535-9001
      (B) TELEFAX: 619-535-8949
(2) INFORMATION FOR SEQ ID NO:1:
   (i) SEQUENCE CHARACTERISTICS:
      (A) LENGTH: 7445 base pairs
      (B) TYPE: nucleic acid
      (C) STRANDEDNESS: both
      (D) TOPOLOGY: circular
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 7317 base pairs
      (B) TYPE: nucleic acid
      (C) STRANDEDNESS: both
      (D) TOPOLOGY: circular
   (xi) SEQUENCE DESCRIPTION: SEQ ID NO:2:
   AATGCTACTA CTATTAGTAG AATTGATGCC ACCTTTTCAG CTCGCGCCCC AAATGAAAAT     60
   ATAGCTAAAC AGGTTATTGA CCATTTGCGA AATGTATCTA ATGGTCAAAC TAAATCTACT    120
   CGTTCGCAGA ATTGGGAATC AACTGTTACA TGGAATGAAA CTTCCAGACA CCGTACTTTA    180
   GTTGCATATT TAAAACATGT TGAGCTACAG CACCAGATTC AGCAATTAAG CTCTAAGCCA    240
   TCCGCAAAAA TGACCTCTTA TCAAAAGGAG CAATTAAAGG TACTCTCTAA TCCTGACCTG    300
   TTGGAGTTTG CTTCCGGTCT GGTTCGCTTT GAAGCTCGAA TTAAAACGCG ATATTTGAAG    360
   TCTTTCGGGC TTCCTCTTAA TCTTTTTGAT GCAATCCGCT TTGCTTCTGA CTATAATAGT    420
   CAGGGTAAAG ACCTGATTTT TGATTTATGG TCATTCTCGT TTTCTGAACT GTTTAAAGCA    480
   TTTGAGGGGG ATTCAATGAA TATTTATGAC GATTCCGCAG TATTGGACGC TATCCAGTCT    540
   AAACATTTTA CTATTACCCC CTCTGGCAAA ACTTCTTTTG CAAAAGCCTC TCGCTATTTT    600
   GGTTTTTATC GTCGTCTGGT AAACGAGGGT TATGATAGTG TTGCTCTTAC TATGCCTCGT    660
   AATTCCTTTT GGCGTTATGT ATCTGCATTA GTTGAATGTG GTATTCCTAA ATCTCAACTG    720
   ATGAATCTTT CTACCTGTAA TAATGTTGTT CCGTTAGTTC GTTTTATTAA CGTAGATTTT    780
   TCTTCCCAAC GTCCTGACTG GTATAATGAG CCAGTTCTTA AAATCGCATA AGGTAATTCA    840
   CAATGATTAA AGTTGAAATT AAACCATCTC AAGCCCAATT TACTACTCGT TCTGGTGTTT    900
   CTCGTCAGGG CAAGCCTTAT TCACTGAATG AGCAGCTTTG TTACGTTGAT TTGGGTAATG    960
   AATATCCGGT TCTTGTCAAG ATTACTCTTG ATGAAGGTCA GCCAGCCTAT GCGCCTGGTC   1020
   TGTACACCGT TCATCTGTCC TCTTTCAAAG TTGGTCAGTT CGGTTCCCTT ATGATTGACC   1080
   GTCTGCGCCT CGTTCCGGCT AAGTAACATG GAGCAGGTCG CGGATTTCGA CACAATTTAT   1140
   CAGGCGATGA TACAAATCTC CGTTGTACTT TGTTTCGCGC TTGGTATAAT CGCTGGGGGT   1200
   CAAAGATGAG TGTTTTAGTG TATTCTTTCG CCTCTTTCGT TTTAGGTTGG TGCCTTCGTA   1260
   GTGGCATTAC GTATTTTACC CGTTTAATGG AAACTTCCTC ATGAAAAAGT CTTTAGTCCT   1320
   CAAAGCCTCT GTAGCCGTTG CTACCCTCGT TCCGATGCTG TCTTTCGCTG CTGAGGGTGA   1380
   CGATCCCGCA AAAGCGGCCT TTAACTCCCT GCAAGCCTCA GCGACCGAAT ATATCGGTTA   1440
   TGCGTGGGCG ATGGTTGTTG TCATTGTCGG CGCAACTATC GGTATCAAGC TGTTTAAGAA   1500
   ATTCACCTCG AAAGCAAGCT GATAAACCGA TACAATTAAA GGCTCCTTTT GGAGCCTTTT   1560
   TTTTTGGAGA TTTTCAACGT GAAAAAATTA TTATTCGCAA TTCCTTTAGT TGTTCCTTTC   1620
   TATTCTCACT CCGCTGAAAC TGTTGAAAGT TGTTTAGCAA AACCCCATAC AGAAAATTCA   1680
   TTTACTAACG TCTGGAAAGA CGACAAAACT TTAGATCGTT ACGCTAACTA TGAGGGTTGT   1740
   CTGTGGAATG CTACAGGCGT TGTAGTTTGT ACTGGTGACG AAACTCAGTG TTACGGTACA   1800
   TGGGTTCCTA TTGGGCTTGC TATCCCTGAA AATGAGGGTG GTGGCTCTGA GGGTGGCGGT   1860
   TCTGAGGGTG GCGGTTCTGA GGGTGGCGGT ACTAAACCTC CTGAGTACGG TGATACACCT   1920
   ATTCCGGGCT ATACTTATAT CAACCCTCTC GACGGCACTT ATCCGCCTGG TACTGAGCAA   1980
   AACCCCGCTA ATCCTAATCC TTCTCTTGAG GAGTCTCAGC CTCTTAATAC TTTCATGTTT   2040
   CAGAATAATA GGTTCCGAAA TAGGCAGGGG GCATTAACTG TTTATACGGG CACTGTTACT   2100
   CAAGGCACTG ACCCCGTTAA AACTTATTAC CAGTACACTC CTGTATCATC AAAAGCCATG   2160
   TATGACGCTT ACTGGAACGG TAAATTCAGA GACTGCGCTT TCCATTCTGG CTTTAATGAA   2220
   GATCCATTCG TTTGTGAATA TCAAGGCCAA TCGTCTGACC TGCCTCAACC TCCTGTCAAT   2280
   GCTGGCGGCG GCTCTGGTGG TGGTTCTGGT GGCGGCTCTG AGGGTGGTGG CTCTGAGGGT   2340
   GGCGGTTCTG AGGGTGGCGG CTCTGAGGGA GGCGGTTCCG GTGGTGGCTC TGGTTCCGGT   2400
   GATTTTGATT ATGAAAAGAT GGCAAACGCT AATAAGGGGG CTATGACCGA AAATGCCGAT   2460
   GAAAACGCGC TACAGTCTGA CGCTAAAGGC AAACTTGATT CTGTCGCTAC TGATTACGGT   2520
   GCTGCTATCG ATGGTTTCAT TGGTGACGTT TCCGGCCTTG CTAATGGTAA TGGTGCTACT   2580
   GGTGATTTTG CTGGCTCTAA TTCCCAAATG GCTCAAGTCG GTGACGGTGA TAATTCACCT   2640
   TTAATGAATA ATTTCCGTCA ATATTTACCT TCCCTCCCTC AATCGGTTGA ATGTCGCCCT   2700
   TTTGTCTTTA GCGCTGGTAA ACCATATGAA TTTTCTATTG ATTGTGACAA AATAAACTTA   2760
   TTCCGTGGTG TCTTTGCGTT TCTTTTATAT GTTGCCACCT TTATGTATGT ATTTTCTACG 2820
   TTTGCTAACA TACTGCGTAA TAAGGAGTCT TAATCATGCC AGTTCTTTTG GGTATTCCGT 2880
   TATTATTGCG TTTCCTCGGT TTCCTTCTGG TAACTTTGTT CGGCTATCTG CTTACTTTTC 2940
   TTAAAAAGGG CTTCGGTAAG ATAGCTATTG CTATTTCATT GTTTCTTGCT CTTATTATTG 3000
   GGCTTAACTC AATTCTTGTG GGTTATCTCT CTGATATTAG CGCTCAATTA CCCTCTGACT 3060
   TTGTTCAGGG TGTTCAGTTA ATTCTCCCGT CTAATGCGCT TCCCTGTTTT TATGTTATTC 3120
   TCTCTGTAAA GGCTGCTATT TTCATTTTTG ACGTTAAACA AAAAATCGTT TCTTATTTGG 3180
   ATTGGGATAA ATAATATGGC TGTTTATTTT GTAACTGGCA AATTAGGCTC TGGAAAGACG 3240
   CTCGTTAGCG TTGGTAAGAT TCAGGATAAA ATTGTAGCTG GGTGCAAAAT AGCAACTAAT 3300
   CTTGATTTAA GGCTTCAAAA CCTCCCGCAA GTCGGGAGGT TCGCTAAAAC GCCTCGCGTT 3360
   CTTAGAATAC CGGATAAGCC TTCTATATCT GATTTGCTTG CTATTGGGCG CGGTAATGAT 3420
   TCCTACGATG AAAATAAAAA CGGCTTGCTT GTTCTCGATG AGTGCGGTAC TTGGTTTAAT 3480
   ACCCGTTCTT GGAATGATAA GGAAAGACAG CCGATTATTG ATTGGTTTCT ACATGCTCGT 3540
   AAATTAGGAT GGGATATTAT TTTTCTTGTT CAGGACTTAT CTATTGTTGA TAAACAGGCG 3600
   CGTTCTGCAT TAGCTGAACA TGTTGTTTAT TGTCGTCGTC TGGACAGAAT TACTTTACCT 3660
   TTTGTCGGTA CTTTATATTC TCTTATTACT GGCTCGAAAA TGCCTCTGCC TAAATTACAT 3720
   GTTGGCGTTG TTAAATATGG CGATTCTCAA TTAAGCCCTA CTGTTGAGCG TTGGCTTTAT 3780
   ACTGGTAAGA ATTTGTATAA CGCATATGAT ACTAAACAGG CTTTTTCTAG TAATTATGAT 3840
   TCCGGTGTTT ATTCTTATTT AACGCCTTAT TTATCACACG GTCGGTATTT CAAACCATTA 3900
   AATTTAGGTC AGAAGATGAA GCTTACTAAA ATATATTTGA AAAAGTTTTC ACGCGTTCTT 3960
   TGTCTTGCGA TTGGATTTGC ATCAGCATTT ACATATAGTT ATATAACCCA ACCTAAGCCG 4020
   GAGGTTAAAA AGGTAGTCTC TCAGACCTAT GATTTTGATA AATTCACTAT TGACTCTTCT 4080
   CAGCGTCTTA ATCTAAGCTA TCGCTATGTT TTCAAGGATT CTAAGGGAAA ATTAATTAAT 4140
   AGCGACGATT TACAGAAGCA AGGTTATTCA CTCACATATA TTGATTTATG TACTGTTTCC 4200
   ATTAAAAAAG GTAATTCAAA TGAAATTGTT AAATGTAATT AATTTTGTTT TCTTGATGTT 4260
   TGTTTCATCA TCTTCTTTTG CTCAGGTAAT TGAAATGAAT AATTCGCCTC TGCGCGATTT 4320
   TGTAACTTGG TATTCAAAGC AATCAGGCGA ATCCGTTATT GTTTCTCCCG ATGTAAAAGG 4380
   TACTGTTACT GTATATTCAT CTGACGTTAA ACCTGAAAAT CTACGCAATT TCTTTATTTC 4440
   TGTTTTACGT GCTAATAATT TTGATATGGT TGGTTCAATT CCTTCCATAA TTCAGAAGTA 4500
   TAATCCAAAC AATCAGGATT ATATTGATGA ATTGCCATCA TCTGATAATC AGGAATATGA 4560
   TGATAATTCC GCTCCTTCTG GTGGTTTCTT TGTTCCGCAA AATGATAATG TTACTCAAAC 4620
   TTTTAAAATT AATAACGTTC GGGCAAAGGA TTTAATACGA GTTGTCGAAT TGTTTGTAAA 4680
   GTCTAATACT TCTAAATCCT CAAATGTATT ATCTATTGAC GGCTCTAATC TATTAGTTGT 4740
   TAGTGCACCT AAAGATATTT TAGATAACCT TCCTCAATTC CTTTCTACTG TTGATTTGCC 4800
   AACTGACCAG ATATTGATTG AGGGTTTGAT ATTTGAGGTT CAGCAAGGTG ATGCTTTAGA 4860
   TTTTTCATTT GCTGCTGGCT CTCAGCGTGG CACTGTTGCA GGCGGTGTTA ATACTGACCG 4920
   CCTCACCTCT GTTTTATCTT CTGCTGGTGG TTCGTTCGGT ATTTTTAATG GCGATGTTTT 4980
   AGGGCTATCA GTTCGCGCAT TAAAGACTAA TAGCCATTCA AAAATATTGT CTGTGCCACG 5040
   TATTCTTACG CTTTCAGGTC AGAAGGGTTC TATCTCTGTT GGCCAGAATG TCCCTTTTAT 5100
   TACTGGTCGT GTGACTGGTG AATCTGCCAA TGTAAATAAT CCATTTCAGA CGATTGAGCG 5160
   TCAAAATGTA GGTATTTCCA TGAGCGTTTT TCCTGTTGCA ATGGCTGGCG GTAATATTGT 5220
   TCTGGATATT ACCAGCAAGG CCGATAGTTT GAGTTCTTCT ACTCAGGCAA GTGATGTTAT 5280
   TACTAATCAA AGAAGTATTG CTACAACGGT TAATTTGCGT GATGGACAGA CTCTTTTACT 5340
   CGGTGGCCTC ACTGATTATA AAAACACTTC TCAAGATTCT GGCGTACCGT TCCTGTCTAA 5400
   AATCCCTTTA ATCGGCCTCC TGTTTAGCTC CCGCTCTGAT TCCAACGAGG AAAGCACGTT 5460
   ATACGTGCTC GTCAAAGCAA CCATAGTACG CGCCCTGTAG CGGCGCATTA AGCGCGGCGG 5520
   GTGTGGTGGT TACGCGCAGC GTGACCGCTA CACTTGCCAG CGCCCTAGCG CCCGCTCCTT 5580
   TCGCTTTCTT CCCTTCCTTT CTCGCCACGT TCGCCGGCTT TCCCCGTCAA GCTCTAAATC 5640
   GGGGGCTCCC TTTAGGGTTC CGATTTAGTG CTTTACGGCA CCTCGACCCC AAAAAACTTG 5700
   ATTTGGGTGA TGGTTCACGT AGTGGGCCAT CGCCCTGATA GACGGTTTTT CGCCCTTTGA 5760
   CGTTGGAGTC CACGTTCTTT AATAGTGGAC TCTTGTTCCA AACTGGAACA ACACTCAACC 5820
   CTATCTCGGG CTATTCTTTT GATTTATAAG GGATTTTGCC GATTTCGGAA CCACCATCAA 5880
   ACAGGATTTT CGCCTGCTGG GGCAAACCAG CGTGGACCGC TTGCTGCAAC TCTCTCAGGG 5940
   CCAGGCGGTG AAGGGCAATC AGCTGTTGCC CGTCTCGCTG GTGAAAAGAA AAACCACCCT 6000
   GGCGCCCAAT ACGCAAACCG CCTCTCCCCG CGCGTTGGCC GATTCATTAA TGCAGCTGGC 6060
   ACGACAGGTT TCCCGACTGG AAAGCGGGCA GTGAGCGCAA CGCAATTAAT GTGAGTTAGC 6120
   TCACTCATTA GGCACCCCAG GCTTTACACT TTATGCTTCC GGCTCGTATG TTGTGTGGAA 6180
   TTGTGAGCGG ATAACAATTT CACACGCCAA GGAGACAGTC ATAATGAAAT ACCTATTGCC 6240
   TACGGCAGCC GCTGGATTGT TATTACTCGC TGCCCAACCA GCCATGGCCG AGCTCGTGAT 6300
   GACCCAGACT CCAGATATCC AACAGGAATG AGTGTTAATT CTAGAACGCG TCACTTGGCA 6360
   CTGGCCGTCG TTTTACAACG TCGTGACTGG GAAAACCCTG GCGTTACCCA AGCTTAATCG 6420
   CCTTGCAGAA TTCCCTTTCG CCAGCTGGCG TAATAGCGAA GAGGCCCGCA CCGATCGCCC 6480
   TTCCCAACAG TTGCGCAGCC TGAATGGCGA ATGGCGCTTT GCCTGGTTTC CGGCACCAGA 6540
   AGCGGTGCCG GAAAGCTGGC TGGAGTGCGA TCTTCCTGAG GCCGATACGG TCGTCGTCCC 6600
   CTCAAACTGG CAGATGCACG GTTACGATGC GCCCATCTAC ACCAACGTAA CCTATCCCAT 6660
   TACGGTCAAT CCGCCGTTTG TTCCCACGGA GAATCCGACG GGTTGTTACT CGCTCACATT 6720
   TAATGTTGAT GAAAGCTGGC TACAGGAAGG CCAGACGCGA ATTATTTTTG ATGGCGTTCC 6780
   TATTGGTTAA AAAATGAGCT GATTTAACAA AAATTTAACG CGAATTTTAA CAAAATATTA 6840
   ACGTTTACAA TTTAAATATT TGCTTATACA ATCTTCCTGT TTTTGGGGCT TTTCTGATTA    6900
   TCAACCGGGG TACATATGAT TGACATGCTA GTTTTACGAT TACCGTTCAT CGATTCTCTT    6960
   GTTTGCTCCA GACTCTCAGG CAATGACCTG ATAGCCTTTG TAGATCTCTC AAAAATAGCT    7020
   ACCCTCTCCG GCATTAATTT ATCAGCTAGA ACGGTTGAAT ATCATATTGA TGGTGATTTG    7080
   ACTGTCTCCG GCCTTTCTCA CCCTTTTGAA TCTTTACCTA CACATTAGTC AGGCATTGCA    7140
   TTTAAAATAT ATGAGGGTTC TAAAAATTTT TATCCTTGCG TTGAAATAAA GGCTTCTCCC    7200
   GCAAAAGTAT TACAGGGTCA TAATGTTTTT GGTACAACCG ATTTAGCTTT ATGCTCTGAG    7260
   GCTTTATTGC TTAATTTTGC TAATTCTTTG CCTTGCCTGT ATGATTTATT GGATGTT       7317
(2) INFORMATION FOR SEQ ID NO:3:
   (i) SEQUENCE CHARACTERISTICS:
      (A) LENGTH: 7729 base pairs
      (B) TYPE: nucleic acid
      (C) STRANDEDNESS: both
      (D) TOPOLOGY: circular
   (xi) SEQUENCE DESCRIPTION: SEQ ID NO:3:
(2) INFORMATION FOR SEQ ID NO:4:
   (i) SEQUENCE CHARACTERISTICS:
      (A) LENGTH: 7557 base pairs
      (B) TYPE: nucleic acid
      (C) STRANDEDNESS: both
      (D) TOPOLOGY: circular
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 8118 base pairs
      (B) TYPE: nucleic acid
      (C) STRANDEDNESS: both
      (D) TOPOLOGY: circular
   (xi) SEQUENCE DESCRIPTION: SEQ ID NO:5:
   AATGCTACTA CTATTAGTAG AATTGATGCC ACCTTTTCAG CTCGCGCCCC AAATGAAAAT 60
   ATAGCTAAAC AGGTTATTGA CCATTTGCGA AATGTATCTA ATGGTCAAAC TAAATCTACT 120
   CGTTCGCAGA ATTGGGAATC AACTGTTACA TGGAATGAAA CTTCCAGACA CCGTACTTTA 180
   GTTGCATATT TAAAACATGT TGAGCTACAG CACCAGATTC AGCAATTAAG CTCTAAGCCA 240
   TCTGCAAAAA TGACCTCTTA TCAAAAGGAG CAATTAAAGG TACTCTCTAA TCCTGACCTG 300
   TTGGAGTTTG CTTCCGGTCT GGTTCGCTTT GAAGCTCGAA TTAAAACGCG ATATTTGAAG 360
   TCTTTCGGGC TTCCTCTTAA TCTTTTTGAT GCAATCCGCT TTGCTTCTGA CTATAATAGT 420
   CAGGGTAAAG ACCTGATTTT TGATTTATGG TCATTCTCGT TTTCTGAACT GTTTAAAGCA 480
   TTTGAGGGGG ATTCAATGAA TATTTATGAC GATTCCGCAG TATTGGACGC TATCCAGTCT 540
   AAACATTTTA CTATTACCCC CTCTGGCAAA ACTTCTTTTG CAAAAGCCTC TCGCTATTTT 600
   GGTTTTTATC GTCGTCTGGT AAACGAGGGT TATGATAGTG TTGCTCTTAC TATGCCTCGT 660
   AATTCCTTTT GGCGTTATGT ATCTGCATTA GTTGAATGTG GTATTCCTAA ATCTCAACTG 720
   ATGAATCTTT CTACCTGTAA TAATGTTGTT CCGTTAGTTC GTTTTATTAA CGTAGATTTT 780
   TCTTCCCAAC GTCCTGACTG GTATAATGAG CCAGTTCTTA AAATCGCATA AGGTAATTCA 840
   CAATGATTAA AGTTGAAATT AAACCATCTC AAGCCCAATT TACTACTCGT TCTGGTGTTT 900
   CTCGTCAGGG CAAGCCTTAT TCACTGAATG AGCAGCTTTG TTACGTTGAT TTGGGTAATG 960
   AATATCCGGT TCTTGTCAAG ATTACTCTTG ATGAAGGTCA GCCAGCCTAT GCGCCTGGTC 1020
   TGTACACCGT TCATCTGTCC TCTTTCAAAG TTGGTCAGTT CGGTTCCCTT ATGATTGACC 1080
   GTCTGCGCCT CGTTCCGGCT AAGTAACATG GAGCAGGTCG CGGATTTCGA CACAATTTAT 1140
   CAGGCGATGA TACAAATCTC CGTTGTACTT TGTTTCGCGC TTGGTATAAT CGCTGGGGGT 1200
   CAAAGATGAG TGTTTTAGTG TATTCTTTCG CCTCTTTCGT TTTAGGTTGG TGCCTTCGTA 1260
   GTGGCATTAC GTATTTTACC CGTTTAATGG AAACTTCCTC ATGAAAAAGT CTTTAGTCCT 1320
   CAAAGCCTCT GTAGCCGTTG CTACCCTCGT TCCGATGCTG TCTTTCGCTG CTGAGGGTGA 1380
   CGATCCCGCA AAAGCGGCCT TTAACTCCCT GCAAGCCTCA GCGACCGAAT ATATCGGTTA 1440
   TGCGTGGGCG ATGGTTGTTG TCATTGTCGG CGCAACTATC GGTATCAAGC TGTTTAAGAA 1500
   ATTCACCTCG AAAGCAAGCT GATAAACCGA TACAATTAAA GGCTCCTTTT GGAGCCTTTT   1560
   TTTTTGGAGA TTTTCAACGT GAAAAAATTA TTATTCGCAA TTCCTTTAGT TGTTCCTTTC   1620
   TATTCTCACT CCGCTGAAAC TGTTGAAAGT TGTTTAGCAA AACCCCATAC AGAAAATTCA   1680
   TTTACTAACG TCTGGAAAGA CGACAAAACT TTAGATCGTT ACGCTAACTA TGAGGGTTGT   1740
   CTGTGGAATG CTACAGGCGT TGTAGTTTGT ACTGGTGACG AAACTCAGTG TTACGGTACA   1800
   TGGGTTCCTA TTGGGCTTGC TATCCCTGAA AATGAGGGTG GTGGCTCTGA GGGTGGCGGT   1860
   TCTGAGGGTG GCGGTTCTGA GGGTGGCGGT ACTAAACCTC CTGAGTACGG TGATACACCT   1920
   ATTCCGGGCT ATACTTATAT CAACCCTCTC GACGGCACTT ATCCGCCTGG TACTGAGCAA   1980
   AACCCCGCTA ATCCTAATCC TTCTCTTGAG GAGTCTCAGC CTCTTAATAC TTTCATGTTT   2040
   CAGAATAATA GGTTCCGAAA TAGGCAGGGG GCATTAACTG TTTATACGGG CACTGTTACT   2100
   CAAGGCACTG ACCCCGTTAA AACTTATTAC CAGTACACTC CTGTATCATC AAAAGCCATG   2160
   TATGACGCTT ACTGGAACGG TAAATTCAGA GACTGCGCTT TCCATTCTGG CTTTAATGAA   2220
   GATCCATTCG TTTGTGAATA TCAAGGCCAA TCGTCTGACC TGCCTCAACC TCCTGTCAAT   2280
   GCTGGCGGCG GCTCTGGTGG TGGTTCTGGT GGCGGCTCTG AGGGTGGTGG CTCTGAGGGT   2340
   GGCGGTTCTG AGGGTGGCGG CTCTGAGGGA GGCGGTTCCG GTGGTGGCTC TGGTTCCGGT   2400
   GATTTTGATT ATGAAAAGAT GGCAAACGCT AATAAGGGGG CTATGACCGA AAATGCCGAT   2460
   GAAAACGCGC TACAGTCTGA CGCTAAAGGC AAACTTGATT CTGTCGCTAC TGATTACGGT   2520
   GCTGCTATCG ATGGTTTCAT TGGTGACGTT TCCGGCCTTG CTAATGGTAA TGGTGCTACT   2580
   GGTGATTTTG CTGGCTCTAA TTCCCAAATG GCTCAAGTCG GTGACGGTGA TAATTCACCT   2640
   TTAATGAATA ATTTCCGTCA ATATTTACCT TCCCTCCCTC AATCGGTTGA ATGTCGCCCT   2700
   TTTGTCTTTA GCGCTGGTAA ACCATATGAA TTTTCTATTG ATTGTGACAA AATAAACTTA   2760
   TTCCGTGGTG TCTTTGCGTT TCTTTTATAT GTTGCCACCT TTATGTATGT ATTTTCTACG   2820
   TTTGCTAACA TACTGCGTAA TAAGGAGTCT TAATCATGCC AGTTCTTTTG GGTATTCCGT   2880
   TATTATTGCG TTTCCTCGGT TTCCTTCTGG TAACTTTGTT CGGCTATCTG CTTACTTTTC   2940
   TTAAAAAGGG CTTCGGTAAG ATAGCTATTG CTATTTCATT GTTTCTTGCT CTTATTATTG   3000
   GGCTTAACTC AATTCTTGTG GGTTATCTCT CTGATATTAG CGCTCAATTA CCCTCTGACT   3060
   TTGTTCAGGG TGTTCAGTTA ATTCTCCCGT CTAATGCGCT TCCCTGTTTT TATGTTATTC   3120
   TCTCTGTAAA GGCTGCTATT TTCATTTTTG ACGTTAAACA AAAAATCGTT TCTTATTTGG   3180
   ATTGGGATAA ATAATATGGC TGTTTATTTT GTAACTGGCA AATTAGGCTC TGGAAAGACG   3240
   CTCGTTAGCG TTGGTAAGAT TCAGGATAAA ATTGTAGCTG GGTGCAAAAT AGCAACTAAT   3300
   CTTGATTTAA GGCTTCAAAA CCTCCCGCAA GTCGGGAGGT TCGCTAAAAC GCCTCGCGTT   3360
   CTTAGAATAC CGGATAAGCC TTCTATATCT GATTTGCTTG CTATTGGGCG CGGTAATGAT   3420
   TCCTACGATG AAAATAAAAA CGGCTTGCTT GTTCTCGATG AGTGCGGTAC TTGGTTTAAT   3480
   ACCCGTTCTT GGAATGATAA GGAAAGACAG CCGATTATTG ATTGGTTTCT ACATGCTCGT   3540
   AAATTAGGAT GGGATATTAT TTTTCTTGTT CAGGACTTAT CTATTGTTGA TAAACAGGCG 3600
   CGTTCTGCAT TAGCTGAACA TGTTGTTTAT TGTCGTCGTC TGGACAGAAT TACTTTACCT 3660
   TTTGTCGGTA CTTTATATTC TCTTATTACT GGCTCGAAAA TGCCTCTGCC TAAATTACAT 3720
   GTTGGCGTTG TTAAATATGG CGATTCTCAA TTAAGCCCTA CTGTTGAGCG TTGGCTTTAT 3780
   ACTGGTAAGA ATTTGTATAA CGCATATGAT ACTAAACAGG CTTTTTCTAG TAATTATGAT 3840
   TCCGGTGTTT ATTCTTATTT AACGCCTTAT TTATCACACG GTCGGTATTT CAAACCATTA 3900
   AATTTAGGTC AGAAGATGAA GCTTACTAAA ATATATTTGA AAAAGTTTTC ACGCGTTCTT 3960
   TGTCTTGCGA TTGGATTTGC ATCAGCATTT ACATATAGTT ATATAACCCA ACCTAAGCCG 4020
   GAGGTTAAAA AGGTAGTCTC TCAGACCTAT GATTTTGATA AATTCACTAT TGACTCTTCT 4080
   CAGCGTCTTA ATCTAAGCTA TCGCTATGTT TTCAAGGATT CTAAGGGAAA ATTAATTAAT 4140
   AGCGACGATT TACAGAAGCA AGGTTATTCA CTCACATATA TTGATTTATG TACTGTTTCC 4200
   ATTAAAAAAG GTAATTCAAA TGAAATTGTT AAATGTAATT AATTTTGTTT TCTTGATGTT 4260
   TGTTTCATCA TCTTCTTTTG CTCAGGTAAT TGAAATGAAT AATTCGCCTC TGCGCGATTT 4320
   TGTAACTTGG TATTCAAAGC AATCAGGCGA ATCCGTTATT GTTTCTCCCG ATGTAAAAGG 4380
   TACTGTTACT GTATATTCAT CTGACGTTAA ACCTGAAAAT CTACGCAATT TCTTTATTTC 4440
   TGTTTTACGT GCTAATAATT TTGATATGGT TGGTTCAATT CCTTCCATAA TTCAGAAGTA 4500
   TAATCCAAAC AATCAGGATT ATATTGATGA ATTGCCATCA TCTGATAATC AGGAATATGA 4560
   TGATAATTCC GCTCCTTCTG GTGGTTTCTT TGTTCCGCAA AATGATAATG TTACTCAAAC 4620
   TTTTAAAATT AATAACGTTC GGGCAAAGGA TTTAATACGA GTTGTCGAAT TGTTTGTAAA 4680
   GTCTAATACT TCTAAATCCT CAAATGTATT ATCTATTGAC GGCTCTAATC TATTAGTTGT 4740
   TAGTGCACCT AAAGATATTT TAGATAACCT TCCTCAATTC CTTTCTACTG TTGATTTGCC 4800
   AACTGACCAG ATATTGATTG AGGGTTTGAT ATTTGAGGTT CAGCAAGGTG ATGCTTTAGA 4860
   TTTTTCATTT GCTGCTGGCT CTCAGCGTGG CACTGTTGCA GGCGGTGTTA ATACTGACCG 4920
   CCTCACCTCT GTTTTATCTT CTGCTGGTGG TTCGTTCGGT ATTTTTAATG GCGATGTTTT 4980
   AGGGCTATCA GTTCGCGCAT TAAAGACTAA TAGCCATTCA AAAATATTGT CTGTGCCACG 5040
   TATTCTTACG CTTTCAGGTC AGAAGGGTTC TATCTCTGTT GGCCAGAATG TCCCTTTTAT 5100
   TACTGGTCGT GTGACTGGTG AATCTGCCAA TGTAAATAAT CCATTTCAGA CGATTGAGCG 5160
   TCAAAATGTA GGTATTTCCA TGAGCGTTTT TCCTGTTGCA ATGGCTGGCG GTAATATTGT 5220
   TCTGGATATT ACCAGCAAGG CCGATAGTTT GAGTTCTTCT ACTCAGGCAA GTGATGTTAT 5280
   TACTAATCAA AGAAGTATTG CTACAACGGT TAATTTGCGT GATGGACAGA CTCTTTTACT 5340
   CGGTGGCCTC ACTGATTATA AAAACACTTC TCAAGATTCT GGCGTACCGT TCCTGTCTAA 5400
   AATCCCTTTA ATCGGCCTCC TGTTTAGCTC CCGCTCTGAT TCCAACGAGG AAAGCACGTT 5460
   ATACGTGCTC GTCAAAGCAA CCATAGTACG CGCCCTGTAG CGGCGCATTA AGCGCGGCGG 5520
   GTGTGGTGGT TACGCGCAGC GTGACCGCTA CACTTGCCAG CGCCCTAGCG CCCGCTCCTT 5580
   TCGCTTTCTT CCCTTCCTTT CTCGCCACGT TCGCCGGCTT TCCCCGTCAA GCTCTAAATC 5640
   GGGGGCTCCC TTTAGGGTTC CGATTTAGTG CTTTACGGCA CCTCGACCCC AAAAAACTTG 5700
   ATTTGGGTGA TGGTTCACGT AGTGGGCCAT CGCCCTGATA GACGGTTTTT CGCCCTTTGA 5760
   CGTTGGAGTC CACGTTCTTT AATAGTGGAC TCTTGTTCCA AACTGGAACA ACACTCAACC 5820
   CTATCTCGGG CTATTCTTTT GATTTATAAG GGATTTTGCC GATTTCGGAA CCACCATCAA 5880
   ACAGGATTTT CGCCTGCTGG GGCAAACCAG CGTGGACCGC TTGCTGCAAC TCTCTCAGGG 5940
   CCAGGCGGTG AAGGGCAATC AGCTGTTGCC CGTCTCGCTG GTGAAAAGAA AAACCACCCT 6000
   GGCGCCCAAT ACGCAAACCG CCTCTCCCCG CGCGTTGGCC GATTCATTAA TGCAGCTGGC 6060
   ACGACAGGTT TCCCGACTGG AAAGCGGGCA GTGAGCGCAA CGCAATTAAT GTGAGTTAGC 6120
   TCACTCATTA GGCACCCCAG GCTTTACACT TTATGCTTCC GGCTCGTATG TTGTGTGGAA 6180
   TTGTGAGCGG ATAACAATTT CACACGCCAA GGAGACAGTC ATAATGAAAT ACCTATTGCC 6240
   TACGGCAGCC GCTGGATTGT TATTACTCGC TGCCCAACCA GCCATGGCCG AGCTCTTCCC 6300
   GCCATCTGAT GAGCAGTTGA AATCTGGAAC TGCCTCTGTT GTGTGCCTGC TGAATAACTT 6360
   CTATCCCAGA GAGGCCAAAG TACAGTGGAA GGTGGATAAC GCCCTCCAAT CGGGTAACTC 6420
   CCAGGAGAGT GTCACAGAGC AGGACAGCAA GGACAGCACC TACAGCCTCA GCAGCACCCT 6480
   GACGCTGAGC AAAGCAGACT ACGAGAAACA CAAAGTCTAC GCCTGCGAAG TCACCCATCA 6540
   GGGCCTGAGC TCGCCCGTCA CAAAGAGCTT CAACAGGGGA GAGTGTTCTA GAACGCGTCA 6600
   CTTGGCACTG GCCGTCGTTT TACAACGTCG TGACTGGGAA AACCCTGGCG TTACCCAAGC 6660
   TTTGTACATG GAGAAAATAA AGTGAAACAA AGCACTATTG CACTGGCACT CTTACCGTTA 6720
   CTGTTTACCC CTGTGGCAAA AGCCGCCTCC ACCAAGGGCC CATCGGTCTT CCCCCTGGCA 6780
   CCCTCCTCCA AGAGCACCTC TGGGGGCACA GCGGCCCTGG GCTGCCTGGT CAAGACTAAT 6840
   TCCCCGAACC GGTGACGGTG TCGTGGAACT CAGGCGCCCT GACCAGCGGC GTGCACACCT 6900
   TCCCGGCTGT CCTACAGTCC TCAGGACTCT ACTCCCTCAG CAGCGTGGTG ACCGTGCCCT 6960
   CCAGCAGCTT GGGCACCCAG ACCTACATCT GCAACGTGAA TCACAAGCCC AGCAACACCA 7020
   AGGTGGACAA GAAAGCAGAG CCCAAATCTT GTACTAGTGG ATCCTACCCG TACGACGTTC 7080
   CGGACTACGC TTCTTAGGCT GAAGGCGATG ACCCTGCTAA GGCTGCATTC AATAGTTTAC 7140
   AGGCAAGTGC TACTGAGTAC ATTGGCTACG CTTGGGCTAT GGTAGTAGTT ATAGTTGGTG 7200
   CTACCATAGG GATTAAATTA TTCAAAAAGT TTACGAGCAA GGCTTCTTAA GCAATAGCGA 7260
   AGAGGCCCGC ACCGATCGCC CTTCCCAACA GTTGCGCAGC CTGAATGGCG AATGGCGCTT 7320
   TGCCTGGTTT CCGGCACCAG AAGCGGTGCC GGAAAGCTGG CTGGAGTGCG ATCTTCCTGA 7380
   GGCCGATACG GTCGTCGTCC CCTCAAACTG GCAGATGCAC GGTTACGATG CGCCCATCTA 7440
   CACCAACGTA ACCTATCCCA TTACGGTCAA TCCGCCGTTT GTTCCCACGG AGAATCCGAC 7500
   GGGTTGTTAC TCGCTCACAT TTAATGTTGA TGAAAGCTGG CTACAGGAAG GCCAGACGCG 7560
   AATTATTTTT GATGGCGTTC CTATTGGTTA AAAAATGAGC TGATTTAACA AAAATTTAAC 7620
   GCGAATTTTA ACAAAATATT AACGTTTACA ATTTAAATAT TTGCTTATAC AATCTTCCTG    7680
   TTTTTGGGGC TTTTCTGATT ATCAACCGGG GTACATATGA TTGACATGCT AGTTTTACGA    7740
   TTACCGTTCA TCGATTCTCT TGTTTGCTCC AGACTCTCAG GCAATGACCT GATAGCCTTT    7800
   GTAGATCTCT CAAAAATAGC TACCCTCTCC GGCATTAATT TATCAGCTAG AACGGTTGAA    7860
   TATCATATTG ATGGTGATTT GACTGTCTCC GGCCTTTCTC ACCCTTTTGA ATCTTTACCT    7920
   ACACATTACT CAGGCATTGC ATTTAAAATA TATGAGGGTT CTAAAAATTT TTATCCTTGC    7980
   GTTGAAATAA AGGCTTCTCC CGCAAAAGTA TTACAGGGTC ATAATGTTTT TGGTACAACC    8040
   GATTTAGCTT TATGCTCTGA GGCTTTATTG CTTAATTTTG CTAATTCTTT GCCTTGCCTG    8100
   TATGATTTAT TGGACGTT                                                  8118
(2) INFORMATION FOR SEQ ID NO:6:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ix) FEATURE:
      (A) NAME/KEY: misc_difference
      (B) LOCATION: replace(5, "")
      (D) OTHER INFORMATION: /note= "S REPRESENTS EQUAL MIXTURE OF G AND C"
   (ix) FEATURE:
      (A) NAME/KEY: misc_difference
      (B) LOCATION: replace(6, "")
      (D) OTHER INFORMATION: /note= "M REPRESENTS EQUAL MIXTURE OF A AND C"
   (ix) FEATURE:
      (A) NAME/KEY: misc_difference
      (B) LOCATION: replace (8, "")
      (D) OTHER INFORMATION: /note= "R REPRESENTS EQUAL MIXTURE OF A AND G"
   (ix) FEATURE:
      (A) NAME/KEY: misc_difference
      (B) LOCATION: replace(11, "")
      (D) OTHER INFORMATION: /note= "K REPRESENTS EQUAL MIXTURE OF G AND T"
   (ix) FEATURE:
      (A) NAME/KEY: misc_difference
      (B) LOCATION: replace(20, "")
      (D) OTHER INFORMATION: /note= "W REPRESENTS EQUAL MIXTURE OF A AND T"
   (xi) SEQUENCE DESCRIPTION: SEQ ID NO:6:
   AGGTSMARCT KCTCGAGTCW GG 22
(2) INFORMATION FOR SEQ ID NO:7:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:7:
   AGGTCCAGCT GCTCGAGTCT GG 22
(2) INFORMATION FOR SEQ ID NO:8:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:8:
   AGGTCCAGCT GCTCGAGTCA GG 22
(2) INFORMATION FOR SEQ ID NO:9:
   (i) SEQUENCE CHARACTERISTICS:
      A) LENGTH: 22 base pairs
      B) TYPE nucleic acid
      (C) STRANDEDNESS: single
      (D) TOPOLOGY: linear
   (xi) SEQUENCE DESCRIPTION: SEQ ID NO:9:
(2) INFORMATION FOR SEQ ID NO:10:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:10:
   AGGTCCAGCT TCTCGAGTCA GG 22
(2) INFORMATION FOR SEQ ID NO:11:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:11:
   AGGTCCAACT GCTCGAGTCT GG 22
(2) INFORMATION FOR SEQ ID NO:12:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:12:
   AGGTCCAACT GCTCGAGTCA GG 22
(2) INFORMATION FOR SEQ ID NO:13:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:13:
   AGGTCCAACT TCTCGAGTCT GG 22
(2) INFORMATION FOR SEQ ID NO:14:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:14:
   AGGTCCAACT TCTCGAGTCA GG 22
(2) INFORMATION FOR SEQ ID NO:15:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ix) FEATURE:
      (A) NAME/KEY: misc difference
      (B) LOCATION: replace(5..6, "")
      (D) OTHER INFORMATION: /note= "N=INOSINE"
   (ix) FEATURE:
      (A) NAME/KEY: misc_difference
      (B) LOCATION: replace(8, "")
      (D) OTHER INFORMATION: /note= "N-INOSINE"
   (ix) FEATURE:
      (A) NAME/KEY: misc difference
      (B) LOCATION: replace(11, "")
      (D) OTHER INFORMATION: /note= "N=INOSINE"
   (ix) FEATURE:
      (A) NAME/KEY: misc_difference
      (B) LOCATION: replace(20, "")
      (D) OTHER INFORMATION: /note= "W REPRESENTS EQUAL MIXTURE OF A AND T"
   (xi) SEQUENCE DESCRIPTION: SEQ ID NO:15:
   AGGTNNANCT NCTCGAGTCW GG 22
(2) INFORMATION FOR SEQ ID NO:16:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (xi) SEQUENCE DESCRIPTION: SEQ ID NO:16:
   CTATTAACTA GTAACGGTAA CAGTGGTGCC TTGCCCCA 38
(2) INFORMATION FOR SEQ ID NO:17:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:17:
   AGGCTTACTA GTACAATCCC TGGGCACAAT 30
(2) INFORMATION FOR SEQ ID NO:18:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:18:
   CCAGTTCCGA GCTCGTTGTG ACTCAGGAAT CT 32
(2) INFORMATION FOR SEQ ID NO:19:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:19:
   CCAGTTCCGA GCTCGTGTTG ACGCAGCCGC CC 32
(2) INFORMATION FOR SEQ ID NO:20:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:20:
   CCAGTTCCGA GCTCGTGCTC ACCCAGTCTC CA 32
(2) INFORMATION FOR SEQ ID NO:21:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:21:
   CCAGTTCCGA GCTCCAGATG ACCCAGTCTC CA 32
(2) INFORMATION FOR SEQ ID NO:22:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:22:
   CCAGATGTGA GCTCGTGATG ACCCAGACTC CA 32
(2) INFORMATION FOR SEQ ID NO:23:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:23:
   CCAGATGTGA GCTCGTCATG ACCCAGTCTC CA 32
(2) INFORMATION FOR SEQ ID NO:24:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:24:
   CCAGTTCCGA GCTCGTGATG ACACAGTCTC CA 32
(2) INFORMATION FOR SEQ ID NO:25:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:25:
   GCAGCATTCT AGAGTTTCAG CTCCAGCTTG CC 32
(2) INFORMATION FOR SEQ ID NO:26:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:26:
   GCGCCGTCTA GAATTAACAC TCATTCCTGT TGAA 34
(2) INFORMATION FOR SEQ ID NO:27:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (xi) SEQUENCE DESCRIPTION: SEQ ID NO:27:
   GATCCTAGGC TGAAGGCGAT GACCCTGCTA AGGCTGC 37
(2) INFORMATION FOR SEQ ID NO:28:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:28:
   ATTCAATAGT TTACAGGCAA GTGCTACTGA GTACA 35
(2) INFORMATION FOR SEQ ID NO:29:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:29:
   TTGGCTACGC TTGGGCTATG GTAGTAGTTA TAGTT 35
(2) INFORMATION FOR SEQ ID NO:30:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:30:
   GGTGCTACCA TAGGGATTAA ATTATTCAAA AAGTT 35
(2) INFORMATION FOR SEQ ID NO:31:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:31:
   TACGAGCAAG GCTTCTTA 18
(2) INFORMATION FOR SEQ ID NO:32:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:32:
   AGCTTAAGAA GCCTTGCTCG TAAACTTTTT GAATAATTT 39
(2) INFORMATION FOR SEQ ID NO:33:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:33:
   AATCCCTATG GTAGCACCAA CTATAACTAC TACCAT 36
(2) INFORMATION FOR SEQ ID NO:34:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:34:
   AGCCCAAGCG TAGCCAATGT ACTCAGTAGC ACTTG 35
(2) INFORMATION FOR SEQ ID NO:35:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO:35:
   CCTGTAAACT ATTGAATGCA GCCTTAGCAG GGTC 34
(2) INFORMATION FOR SEQ ID NO:36:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (xi) SEQUENCE DESCRIPTION: SEQ ID NO:36:
   ATCGCCTTCA GCCTAG 16
(2) INFORMATION FOR SEQ ID NO:37:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:37:
   CATTTTTGCA GATGGCTTAG A 21
(2) INFORMATION FOR SEQ ID NO:38:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO:38:
   TAGCATTAAC GTCCAATA 18
(2) INFORMATION FOR SEQ ID NO:39:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (xi) SEQUENCE DESCRIPTION: SEQ ID NO:39:
   ATATATTTTA GTAAGCTTCA TCTTCT 26
(2) INFORMATION FOR SEQ ID NO:40:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:40:
   GACAAAGAAC GCGTGAAAAC TTT 23
(2) INFORMATION FOR SEQ ID NO:41:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (xi) SEQUENCE DESCRIPTION: SEQ ID NO:41:
   GCGGGCCTCT TCGCTATTGC TTAAGAAGCC TTGCT 35
(2) INFORMATION FOR SEQ ID NO:42:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (xi) SEQUENCE DESCRIPTION: SEQ ID NO:42:
   AAACGACGGC CAGTGCCAAG TGACGCGTGT GAAATTGTTA TCC 43
(2) INFORMATION FOR SEQ ID NO:43:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (xi) SEQUENCE DESCRIPTION: SEQ ID NO:43:
   GGCGAAAGGG AATTCTGCAA GGCGATTAAG CTTGGGTAAC GCC 43
(2) INFORMATION FOR SEQ ID NO:44:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:44:
   GGCGTTACCC AAGCTTTGTA CATGGAGAAA ATAAAG 36
(2) INFORMATION FOR SEQ ID NO:45:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO:45:
   TGAAACAAAG CACTATTGCA CTGGCACTCT TACCGTTACC GT 42
(2) INFORMATION FOR SEQ ID NO:46:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO:46:
   TACTGTTTAC CCCTGTGACA AAAGCCGCCC AGGTCCAGCT GC 42
(2) INFORMATION FOR SEQ ID NO:47:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (xi) SEQUENCE DESCRIPTION: SEQ ID NO:47:
   TCGAGTCAGG CCTATTGTGC CCAGGGATTG TACTAGTGGA TCCG 44
(2) INFORMATION FOR SEQ ID NO:48:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear.
   (xi) SEQUENCE DESCRIPTION: SEQ ID NO:48:
   TGGCGAAAGG GAATTCGGAT CCACTAGTAC AATCCCTG 38
(2) INFORMATION FOR SEQ ID NO:49:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO:49:
   GGCACAATAG GCCTGACTCG AGCAGCTGGA CCAGGGCGGC TT 42
(2) INFORMATION FOR SEQ ID NO:50:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO:50:
   TTGTCACAGG GGTAAACAGT AACGGTAACG GTAAGTGTGC CA 42
(2) INFORMATION FOR SEQ ID NO:51:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO:51:
   GTGCAATAGT GCTTTGTTTC ACTTTATTTT CTCCATGTAC AA 42
(2) INFORMATION FOR SEQ ID NO:52:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:52:
   TAACGGTAAG AGTGCCAGTG C 21
(2) INFORMATION FOR SEQ ID NO:53:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:53:
   CACCTTCATG AATTCGGCAA GGAGACAGTC AT 32
(2) INFORMATION FOR SEQ ID NO:54:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (xi) SEQUENCE DESCRIPTION: SEQ ID NO:54:
   AATTCGCCAA GGAGACAGTC AT 22
(2) INFORMATION FOR SEQ ID NO:55:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:55:
   AATGAAATAC CTATTGCCTA CGGCAGCCGC TGGATTGTT 39
(2) INFORMATION FOR SEQ ID NO:56:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:56:
   ATTACTCGCT GCCCAACCAG CCATGGCCGA GCTCGTGAT 39
(2) INFORMATION FOR SEQ ID NO:57:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:57:
   GACCCAGACT CCAGATATCC AACAGGAATG AGTGTTAAT 39
(2) INFORMATION FOR SEQ ID NO:58:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (xi) SEQUENCE DESCRIPTION: SEQ ID NO:58:
   TCTAGAACGC GTC 13
(2) INFORMATION FOR SEQ ID NO:59:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION: SEQ ID NO:59:
   TTCAGGTTGA AGCTTACGCG TTCTAGAATT AACACTCATT CCTGT 45
(2) INFORMATION FOR SEQ ID NO:60:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:60:
   TGGATATCTG GAGTCTGGGT CATCACGAGC TCGGCCATG 39
(2) INFORMATION FOR SEQ ID NO:61:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:61:
   GCTGGTTGGG CAGCGAGTAA TAACAATCCA GCGGCTGCC 39
(2) INFORMATION FOR SEQ ID NO:62:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (xi) SEQUENCE DESCRIPTION: SEQ ID NO:62:
   GTAGGCAATA GGTATTTCAT TATGACTGTC CTTGGCG 37
(2) INFORMATION FOR SEQ ID NO:63:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:63:
   TGACTGTCTC CTTGGCGTGT GAAATTGTTA 30
(2) INFORMATION FOR SEQ ID NO:64:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:64:
   TAACACTCAT TCCGGATGGA ATTCTGGAGT CTGGGT 36
(2) INFORMATION FOR SEQ ID NO:65:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:65:
   GCCAGTGCCA AGTGACGCGT TCTA 24
(2) INFORMATION FOR SEQ ID NO:66:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (xi) SEQUENCE DESCRIPTION: SEQ ID NO:66:
   ATATATTTTA GTAAGCTTCA TCTTCT 26
(2) INFORMATION FOR SEQ ID NO:67:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:67:
   GACAAAGAAC GCGTGAAAAC TTT 23
(2) INFORMATION FOR SEQ ID NO:68:
   (i) SEQUENCE CHARACTERISTICS:
      (A) LENGTH: 76 base pairs
      (B) TYPE: nucleic acid
      (C) STRANDEDNESS: single
      (D) TOPOLOGY: linear
   (xi) SEQUENCE DESCRIPTION: SEQ ID NO:68:
   CTGAACCTGT CTGGGACCAC AGTTGATGCT ATAGGATCAG ATCTAGAATT CATTTAGAGA 60
   CTGGCCTGGC TTCTGC 76
(2) INFORMATION FOR SEQ ID NO:69:
   (i) SEQUENCE CHARACTERISTICS:
      (A) LENGTH: 80 base pairs
      (B) TYPE: nucleic acid
      (C) STRANDEDNESS: single
      (D) TOPOLOGY: linear
   (xi) SEQUENCE DESCRIPTION: SEQ ID NO:69:
   TCGACCGTTG GTAGGAATAA TGCAATTAAT GGAGTAGCTC TAAATTCAGA ATTCATCTAC 60
   ACCCAGTGCA TCCAGTAGCT 80
(2) INFORMATION FOR SEQ ID NO:70:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (xi) SEQUENCE DESCRIPTION: SEQ ID NO:70:
   GGTAAACAGT AACGGTAAGA GTGCCAG 27
(2) INFORMATION FOR SEQ ID NO:71:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (xi) SEQUENCE DESCRIPTION: SEQ ID NO:71:
   CGCCTTCAGC CTAAGAAGCG TAGTCCGGAA CGTCGTACGG GTAGGATCCA CTAG 54
(2) INFORMATION FOR SEQ ID NO:72:
   (i) SEQUENCE CHARACTERISTICS:
      (A) LENGTH: 41 base pairs
      (B) TYPE: nucleic acid
      (C) STRANDEDNESS: single
      (D) TOPOLOGY: linear
   (xi) SEQUENCE DESCRIPTION: SEQ ID NO:72:
   CACCGGTTCG GGGAATTAGT CTTGACCAGG CAGCCCAGGG C 41
(2) INFORMATION FOR SEQ ID NO:73:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (xi) SEQUENCE DESCRIPTION: SEQ ID NO:73:
   ATTCCACACA TTATACGAGC CGGAAGCATA AAGTGTCAAG CCTGGGGTGC C 51
(2) INFORMATION FOR SEQ ID NO:74:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO:74:
   CTGCTCATCA GATGGCGGGA AGAGCTCGGC CATGGCTGGT TG 42
(2) INFORMATION FOR SEQ ID NO:75:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO:75:
   GAACAGAGTG ACCGAGGGGG CGAGCTCGGC CATGGCTGGT TG                    42

## Description

### BACKGROUND OF THE INVENTION

This invention relates generally to recombinant expression of heteromeric receptors and, more particularly, to expression of such receptors on the surface of filamentous bacteriophage.

Antibodies are heteromeric receptors generated by a vertebrates organism's immune system which bind to an antigen. The molecules are composed of two heavy and two light chains disulfide bonded together. Antibodies have the appearance of a "Y" - shaped structure and the antigen binding portion being located at the end of both short arms of the Y. The region on the heavy and light chain polypeptides which corresponds to the antigen binding portion is known as variable region. The differences between antibodies within this region are primarily responsible for the variation in binding specificities between antibody molecules. The binding specificities are a composite of the antigen interactions with both heavy and light chain polypeptides.

The immune system has the capability of generating an almost infinite number of different antibodies. Such a large diversity is generated primarily through recombination to form the variable regions of each chain and through differential pairing of heavy and light chains. The ability to mimic the natural immune system and generate antibodies that bind to any desired molecule is valuable because such antibodies can be used for diagnostic and therapeutic purposes.

Until recently, generation of antibodies against a desired molecule was accomplished only through manipulation of natural immune responses. Methods included classical immunization techniques of laboratory animals and monoclonal antibody production. Generation of monoclonal antibodies is laborious and time consuming. It involves a series of different techniques and is only performed on animal cells. Animal cells have relatively long generation times and require extra precautions to be taken compared to procaryotic cells to ensure viability of the cultures.

A method for the generation of a large repertoire of diverse antibody molecules in bacteria has been described, Huse et al., Science, 246, 1275-1281 (1989).
The method uses the bacteriophage lambda as the vector. The lambda vector is a long, linear double-stranded DNA molecule. Production of antibodies using this vector involves the cloning of heavy and light chain populations of DNA sequences into separate vectors. The vectors are subsequently combined randomly to form a single vector which directs the coexpression of heavy and light chains to form antibody fragments. A disadvantage to this method is that undesired combinations of vector portions are brought together when generating the coexpression vector. Although these undesired combinations do not produce viable phage, they do however, result in a significant loss of sequences from the population and, therefore, a loss in diversity of the number of different combinations which can be obtained between heavy and light chains. Additionally, the size of the lambda phage gene is large compared to the genes that encode the antibody segments. This makes the lambda system inherently more difficult to manipulate as compared to other available vector systems.

There thus exists a need for a method to generate diverse populations of heteromeric receptors which mimics the natural immune system, which is fast and efficient and results in only desired combinations without loss of diversity. The present invention satisfies these needs and provides related advantages as well.

### SUMMARY OF THE INVENTION

The invention relates to a plurality of cells containing diverse combinations of first and second DNA sequences encoding first and second polypeptides which form a heteromeric receptor, said heteromeric receptors being expressed on the surface of a cell, preferably one which produces filamentous bacteriophage, such as M13. Vectors, cloning systems and methods of making and screening the heteromeric receptors are also provided.

### BRIEF DESCRIPTION OF THE DRAWINGS

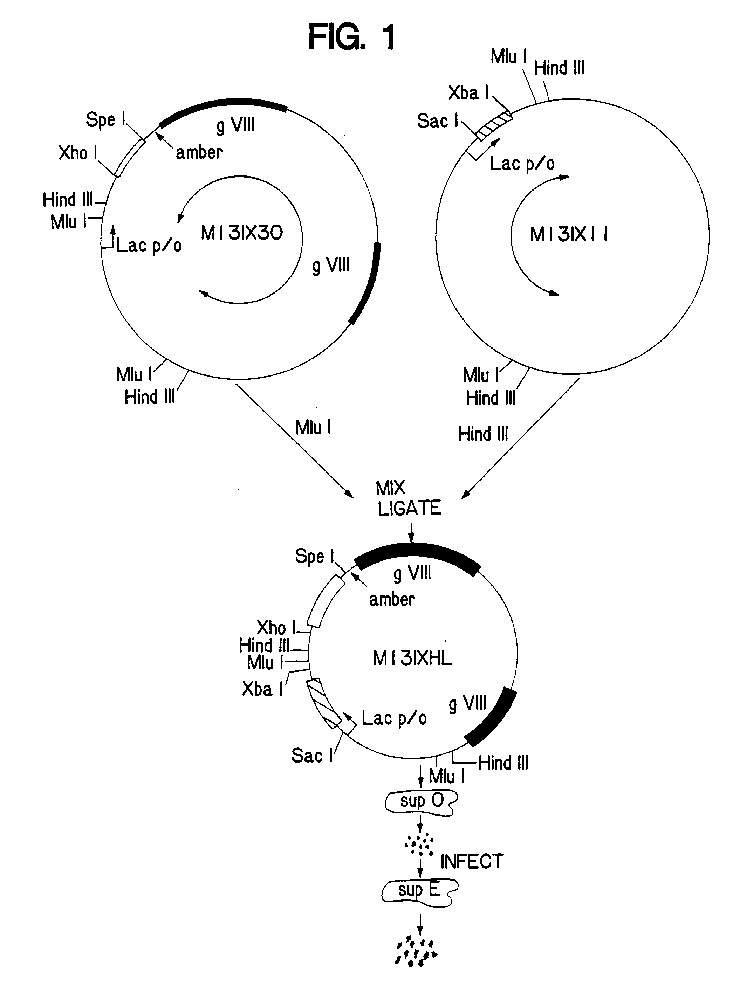

Figure 1 is a schematic diagram of the two vectors used for surface expression library construction from heavy and light chain libraries. M13IX30 (Figure 1A) is the vector used to clone the heavy chain sequences (open box). The single-headed arrow represents the Lac p/o expression sequences and the double-headed arrow represents the portion of M13IX30 which is to be combined with M13IX11. The amber stop codon and relevant restriction sites are also shown. M13IX11 (Figure 1B) is the vector used to clone the light chain sequences (hatched box). Thick lines represent the pseudo-wild type ( gVIII) and wild type (gVIII) gene VIII sequences. The double-headed arrow represents the portion of M13IX11 which is to be combined with M13IX30. Relevant restrictics sites are also shown. Figure 1C shows the joining of vector population from heavy and light chain libraries to form the functional surface expression vector M13IXHL. Figure 1D shows the generation of a surface expression library in a non-suppressor strain and the production of phage. The phage are used to infect a suppressor strain (Figure 1E) for surface expression and screening of the library.

Figure 2 is the nucleotide sequence of M13IX30 (SEQ ID NO: 1).

Figure 3 is the nucleotide sequence of M13IX11 (SEQ ID NO:2).

Figure 4 is the nucleotide sequence of M13IX34 (SEQ ID NO: 3) .

Figure 5 is the nucleotide sequence of M13IX13 (SEQ ID NO: 4).

Figure 6 is the nucleotide sequence of M13IX60 (SEQ ID NO: 5).

### DETAILED DESCRIPTION OF THE INVENTION

This invention is directed to simple and efficient methods to generate a large repertoire of diverse combinations of heteromeric receptors. The method is advantageous in that only proper combinations of vector portions are randomly brought together for the coexpression of different DNA sequences without loss of population size or diversity. The receptors can be expressed on the surface of cells, such as those producing filamentous bacteriophage, which can be screened in large numbers. The nucleic acid sequences encoding the receptors be readily characterized because the filamentous bacteriophage produce single strand DNA for efficient sequencing and mutagenesis methods. The heteromeric receptors so produced are useful in an unlimited number of diagnostic and therapeutic procedures.

In one embodiment, two populations of diverse heavy (Hc) and light (Lc) chain sequences are synthesized by polymerase chain reaction (PCR). These populations are cloned into separate M13-based vector containing elements necessary for expression. The heavy chain vector contains a gene VIII (gVIII) coat protein sequence so that translation of the Hc sequences produces gVIII-Hc fusion proteins. The populations of two vectors are randomly combined such that only the vector portions containing the Hc and Lc sequences are joined into a single circular vector. The combined vector directs the coexpression of both Hc and Lc sequences for assembly of the two polypeptides and surface expression on M13. A mechanism also exists to control the expression of gVIII-Hc fusion proteins during library construction and screening.

As used herein, the term "heteromeric receptors" refers to proteins composed of two or more subunits which together exhibit binding activity toward particular molecule. It is understood that the term includes the subunit fragments so long as assembly of the polypeptides and function of the assembled complex is retained. Heteromeric subunits include, for example, antibodies and fragments thereof such as Fab and (Fab)₂ portions, T cell receptors, integrins, hormone receptors and transmitter receptors.

As used herein, the term "preselected molecule" refers to a molecule which is chosen from a number of choices. The molecule can be, for example, a protein or peptide, or an organic molecule such as a drug. Benzodiazapam is a specific example of a preselected molecule.

As used herein, the term "coexpression" refers to the expression of two or more nucleic acid sequences usually expressed as separate polypeptides. For heteromeric receptors, the coexpressed polypeptides assemble to form the heteromer. Therefore, "expression elements" as used herein, refers to sequences necessary for the transcription, translation, regulation and sorting of the expressed polypeptides which make up the heteromeric receptors. The term also includes the expression of two subunit polypeptides which are linked but are able to assemble into a heteromeric receptor. A specific example of coexpression of linked polypeptides is where Hc and Lc polypeptides are expressed with a flexible peptide or polypeptide linker joining the two subunits into a single chain. The linker is flexible enough to allow association of Hc and Lc portions into a functional Fab fragment.

The invention provides for a composition of matter comprising a plurality of procaryotic cells containing diverse combinations of first and second DNA sequences encoding first and second polypeptides which form a heteromeric receptor exhibiting binding activity toward a preselected molecule, said heteromeric receptors being expressed on the surface of filamentous bacteriophage.

DNA sequences encoding the polypeptides of heteromeric receptors are obtained by methods known to one skilled in the art. Such methods include, for example, cDNA synthesis and polymerase chain reaction (PCR). The need will determine which method or combinations of methods is to be used to obtain the desired populations of sequences. Expression can be performed in any compatible vector/host system. Such systems include, for example, plasmids or phagemids in procaryotes such as E. coli, yeast systems and other eucaryotic systems such as mammalian cells, but will be described herein in context with its presently preferred embodiment, i.e. expression on the surface of filamentous bacteriophage. Filamentous bacteriophage include, for example, M13, fl and fd. Additionally, the heteromeric receptors can also be expressed in soluble or secreted form depending on the need and the vector/host system employed.

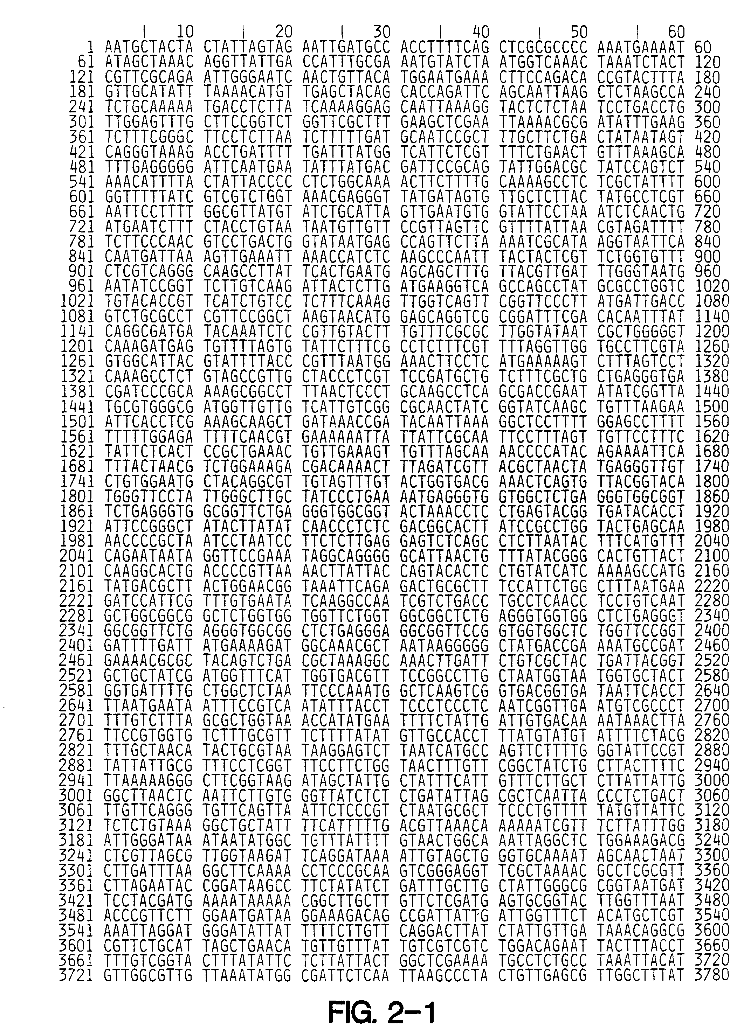

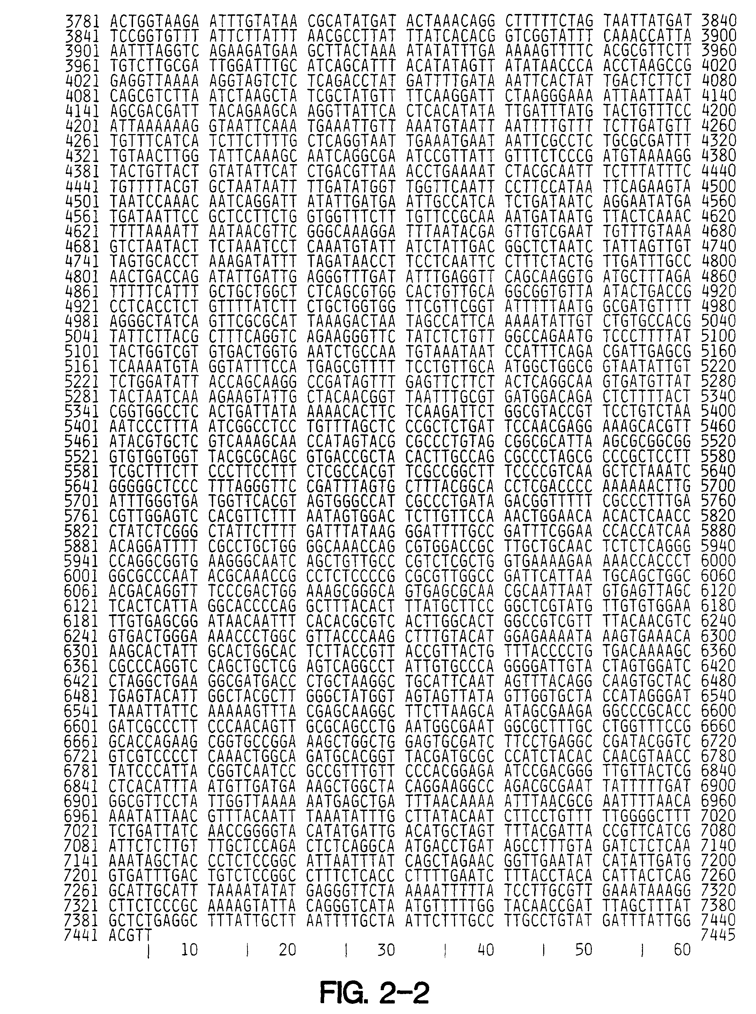

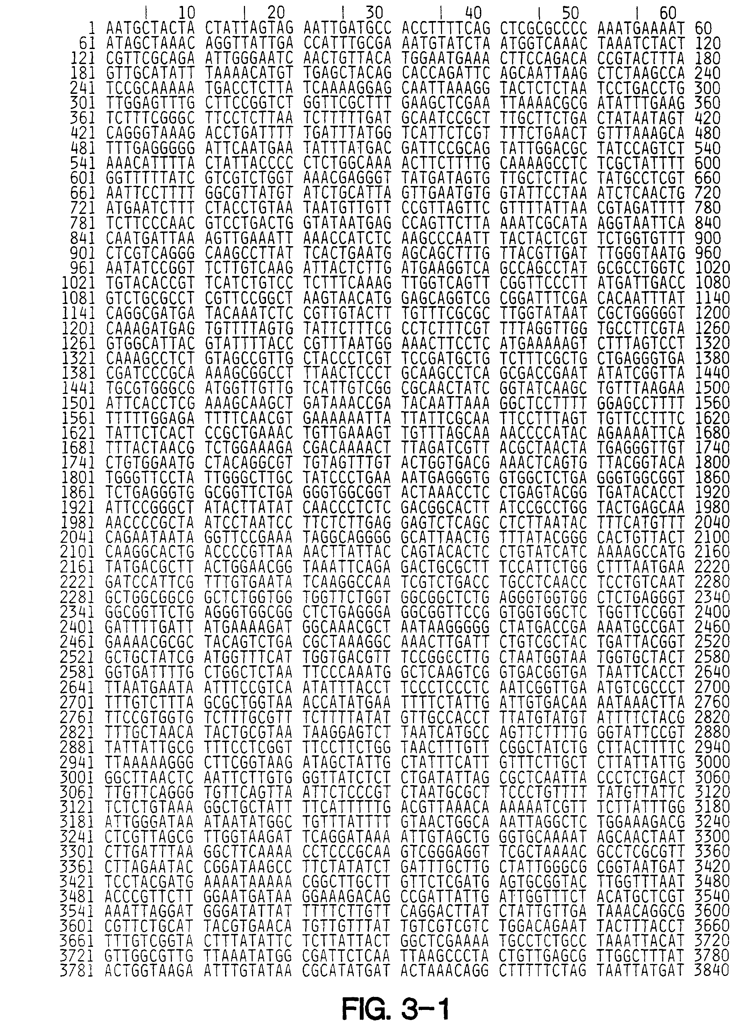

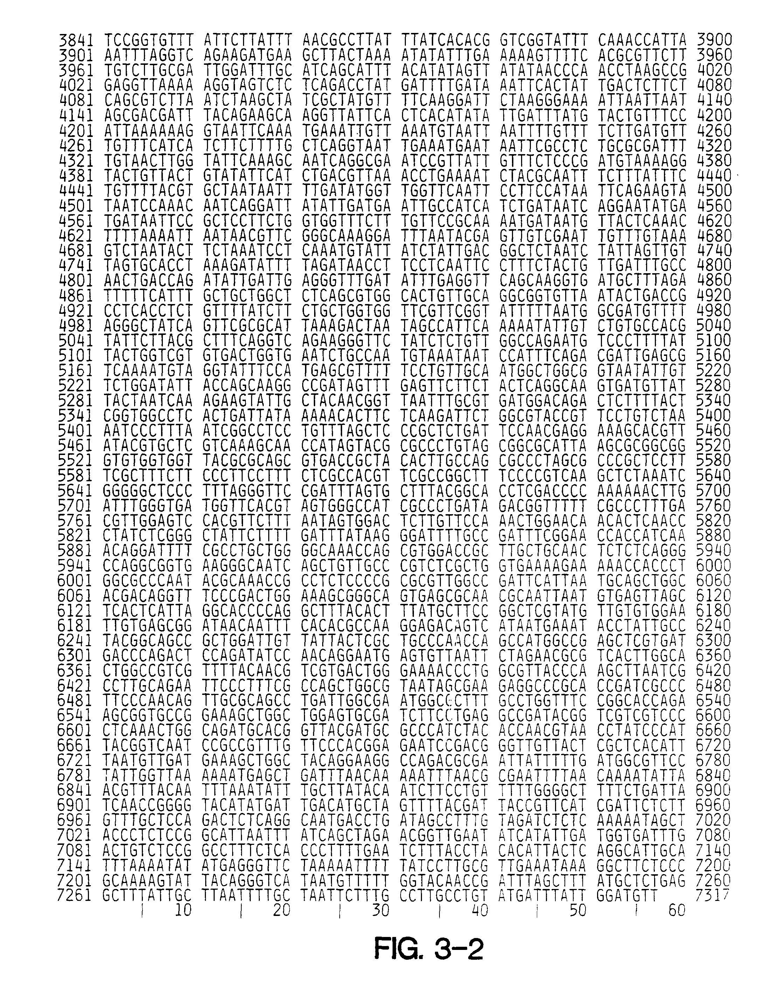

Expression of heteromeric receptors such as antibodies or functional fragments thereof on the surface of M13 can be accomplished, for example, using the vector system shown in Figure 1. Construction of the vectors enabling one of ordinary skill to make them are explicitly set out in Example I. The complete nucleotide sequences are given in Figures 2 and 3 (SEQ ID NOS: 1 and 2). This system produces randomly combined populations of heavy (Hc) and light (Lc) chain antibody fragments functionally linked to expression elements. The Hc polypeptide is produced as a fusion protein with the M13 coat protein encoded by gene VIII. The gVIII-Hc fusion protein therefore anchors the assembled Hc and Lc polypeptides on the surface of M13. The diversity of Hc and Lc combinations obtained by this system can be 5 x 10⁷ or greater. Diversity of less than 5 x 10⁷ can also be obtained and will be determined by the need and type of heteromeric receptor to be expressed.

Populations of Hc and Lc encoding sequences to be combined into a vector for coexpression are each cloned into separate vectors. For the vectors shown in Figure 1, diverse populations of sequences encoding Hc polypeptides are cloned into M13IX30 (SEQ ID NO: 1). Sequences encoding Lc polypeptides are cloned into M13IX11 (SEQ ID NO: 2). The populations are inserted between the Xho I-Spe I or Stu I restriction enzyme sites in M13IX30 and between the Sac I-Xba I or Eco RV sites in M13IX11 (Figures 1A and B, respectively).

The populations of Hc and Lc sequences inserted into the vectors can be synthesized with appropriate restriction recognition sequences flanking opposite ends of the encoding sequences but this is not necessary. The sites allow annealing and ligation in-frame with expression elements of these sequences into a double-stranded vector restricted with the appropriate restriction enzyme. Alternatively, and a preferred embodiment, the Hc and Lc sequences can be inserted into the vector without restriction of the DNA. This method of cloning is beneficial because naturally encoded restriction enzyme sites may be present within the sequences, thus, causing destruction of the sequence when treated with a restriction enzyme. For cloning without restriction, the sequences are treated briefly with a 3' to 5' exonuclease such as T4 DNA polymerase or exonuclease III. A 5' to 3' exonuclease will also accomplish the same function. The protruding 5' termini which remains should be complementary to single-stranded overhangs within the vector which remain after restriction at the cloning site and treatment with exonuclease. The exonuclease treated inserts are annealed with the restricted vector by methods known to one skilled in the art. The exonuclease method decreases background and is easier to perform.

The vector used for Hc populations, M13IX30 (Figure 1A; SEQ ID NO: 1) contains, in addition to expression elements, a sequence encoding the pseudo-wild type gVIII product downstream and in frame with the cloning sites. This gene encodes the wild type M13 gVIII amino acid sequence but has been changed at the nucleotide level to reduce homologous recombination with the wild type gVIII contained on the same vector. The wild type gVIII is present to ensure that at least some functional, non-fusion coat protein will be produced. The inclusion of a wild type gVIII therefore reduces the possibility of non-viable phage production and biological selection against certain peptide fusion proteins. Differential regulation of the two genes can also be used to control the relative ratio of the pseudo and wild type proteins.

Also contained downstream and in frame with the cloning sites is an amber stop codon. The stop codon is located between the inserted Hc sequences and the gVIII sequence and is in frame. As was the function of the wild type gVIII, the amber stop codon also reduces biological selection when combining vector portions to produce functional surface expression vectors. This is accomplished by using a non-suppressor (sup O) host strain because the non-suppressor strains will terminate expression after the Hc sequences but before the pseudo gVIII sequences. Therefore, the pseudo gVIII will essentially never be expressed on the phage surface under these circumstances. Instead, only soluble Hc polypeptides will be produced. Expression in a non-suppressor host strain can be advantageously utilized when one wishes to produce large populations of antibody fragments. Stop codons other than amber, such as opal and ochre, or molecular switches, such as inducible repressor elements, can also be used to unlink peptide expression from surface expression.

The vectc used for Lc populations, M13IX11 (SEQ ID NO: 2), contains necessary expression elements and cloning sites for the Lc sequences, Figure 1B. As with M13IX30, upstream and in frame with the cloning sites is a leader sequence for sorting to the phage surface. Additionally, a ribosome binding site and Lac Z promoter/operator elements are also present for transcription and translation of the DNA sequences.

Both vectors contain two pairs of Mlu I-Hind III restriction enzyme sites (Figures 1A and B) for joining together the Hc and Lc encoding sequences and their associated vector sequences. Mlu I and Hind III are non-compatible restriction sites. The two pairs are symmetrically orientated about the cloning site so that only the vector portions containing the sequences to be expressed are exactly combined into a single vector. The two pairs of sites are oriented identically with respect to one another on both vectors and the DNA between the two sites must be homologous enough between both vectors to allow annealing. This orientation allows cleavage of each circular vector into two portions and combination of essential components within each vector into a single circular vector where the encoded polypeptides can be coexpressed (Figure 1C).

Any two pairs of restriction enzyme sites can be used so long as they are symmetrically orientated about the cloning site and identically orientated on both vectors. The sites within each pair, however, should be non-identical or able to be made differentially recognized as a cleavage substrate. For example, the two pairs of restriction sites contained within the vectors shown in Figure 1 are Mlu I and Hind III. The sites are differentially cleavable by Mlu I and Hind III respectively. One skilled in the art knows how to substitute alternative pairs of restriction enzyme sites for the Mlu I-Hind III pairs described above. Also, instead of two Hind III and two Mlu I sites, a Hind III and Not I site can be paired with a Mlu I and a Sal I site, for example.

The combining step randomly brings together different Hc and Lc encoding sequences within the two diverse populations into a single vector (Figure 1C; M13IXHL). The vector sequences donated from each independent vector, M13IX30 and M13IX11, are necessary for production of viable phage. Also, since the pseudo gVIII sequences are contained in M13IX30, coexpression of functional antibody fragments as Lc associated gVIII-Hc fusion proteins cannot be accomplished on the phage surface until the vector sequences are linked as shown in M13IXHL.

The combining step is performed by restricting each population of Hc and Lc containing vectors with Mlu I and Hind III, respectively. The 3' termini of each restricted vector population is digested with a 3' to 5' exonuclease as described above for inserting sequences into the cloning sites. The vector populations are mixed, allowed to anneal and introduced into an appropriate host. A non-suppressor host (Figure 1D) is preferably used during initial construction of the library to ensure that sequences are not selected against due to expression as fusion proteins. Phage isolated from the library constructed in a non-suppressor strain can be used to infect a suppressor strain for surface expression of antibody fragments.

A method for selecting a heteromeric receptor exhibiting binding activity toward a preselected molecule from a population of diverse heteromeric receptors, comprising: (a) operationally linking to a first vector a first population of diverse DNA sequences encoding a diverse population of first polypeptides, said first vector having two pairs of restriction sites symmetrically oriented about a cloning site; (b) operationally linking to a second vector a second population of diverse DNA sequences encoding a diverse population of second polypeptides, said second vector having two pairs of restriction sites symmetrically oriented about a cloning site in an identical orientation to that of the first vector; (c) combining the vector products of step (a) and (b) under conditions which allow only the operational combination of vector sequences containing said first and second DNA sequences; (d) introducing said population of combined vectors into a compatible host under conditions sufficient for expressing said population of first and second DNA sequences; and (e) determining the heteromeric receptors which bind to said preselected molecule. The invention also provides for determining the nucleic acid sequences encoding such polypeptides as well.

Surface expression of the antibody library is performed in an amber suppressor strain. As described above, the amber stop codon between the Hc sequence and the gVIII sequence unlinks the two components in a non-suppressor strain. Isolating the phage produced from the non-suppressor strain and infecting a suppressor strain will link the Hc sequences to the gVIII sequence during expression (Figure 1E). Culturing the suppressor strain after infection allows the coexpression on the surface of M13 of all antibody species within the library as gVIII fusion proteins (gVIII-Fab fusion proteins). Alternatively, the DNA can be isolated from the non-suppressor strain and then introduced into a suppressor strain to accomplish the same effect.

The level of expression of gVIII-Fab fusion proteins can additionally be controlled at the transcriptional level. Both polypeptides of the gVIII-Fab fusion proteins are under the inducible control of the Lac Z promoter/operator system. Other inducible promoters can work as well and are known by one skilled in the art. For high levels of surface expression, the suppressor library is cultured in an inducer of the Lac Z promoter such as isopropylthio-B-galactoside (IPTG). Inducible control is beneficial because biological selection against non-functional gVIII-Fab fusion proteins can be minimized by culturing the library under non-expressing conditions. Expression can then be induced only at the time of screening to ensure that the entire population of antibodies within the library are accurately represented on the phage surface. Also, this can be used to control the valency of the antibody on the phage surface.

The surface expression library is screened for specific Fab fragments which bind preselected molecules by standard affinity isolation procedures. Such methods include, for example, panning, affinity chromatography and solid phase blotting procedures. Panning as described by Parmley and Smith in Gene 73:305-318 (1988) is preferred because high titers of phage can be screened easily, quickly and in small volumes. Furthermore, this procedure can select minor Fab fragments species within the population, which otherwise would have been undetectable, and amplified to substantially homogenous populations. The selected Fab fragments can be characterized by sequencing the nucleic acids encoding the polypeptides after amplification of the phage population.

The following examples are intended to illustrate but not limit the invention.

### EXAMPLE I

### Construction, Expression and Screening of Antibody Fragments on the Surface of M13

This example shows the synthesis of a diverse population of heavy (Hc) and light (Lc) chain antibody fragments and their expression on the surface of M13 as gene VIII-Fab fusion proteins. The expressed antibodies derive from the random mixing and coexpression of a Hc and Lc pair. Also demonstrated is the isolation and characterization of the expressed Fab fragments which bind benzodiazapam (BDP) and their corresponding nucleotide sequence.

### Isolation of mRNA and PCR Amplification of Antibody Fragments

The surface expression library is constructed from mRNA isolated from a mouse that had been immunized with KLH-coupled benzodiazapam (BDP). BDP was coupled to keyhole limpet hemocyanin (KLH) using the techniques described in Antibodies: A Laboratory Manual, Harlow and Lane, eds., Cold Spring Harbor, New York (1988). Briefly, 10.0 milligrams (mg) of keyhole limpet hemocyanin and 0.5 mg of BDP with a glutaryl spacer arm N-hydroxysuccinimide linker appendages. Coupling was performed as in Jonda et al., Science, 241:1188 (1988), which is incorporated herein by reference. The KLH-BDP conjugate was removed by gel filtration chromatography through Sephadex G-25.

The KLH-BDP conjugate was prepared for injection into mice by adding 100 *µ*g of the conjugate to 250 *µ*l of phosphate buffered saline (PBS). An equal volume of complete Freund's adjuvant was added and emulsified the entire solution for 5 minutes. Mice were injected with 300 *µ*l of the emulsion. Injections were given subcutaneously at several sites using a 21 gauge needle. A second immunization with BDP was given two weeks later. This injection was prepared as follows: 50 *µ*g of BDP was diluted in 250 *µ*l of PBS and an equal volume of alum was mixed with the solution. The mice were injected intraperitoneally with 500 *µ*l of the solution using a 23 gauge needle. One month later the mice were given a final injection of 50 *µ*g of the conjugate diluted to 200 *µ*l in PBS. This injection was given intravenously in the lateral tail vein using a 30 gauge needle. Five days after this final injection the mice were sacrificed and total cellular RNA was isolated from their spleens.

Total RNA was isolated from the spleen of a single mouse immunized as described above by the method of Chomczynski and Sacchi, Anal. Biochem., 162:156-159 (1987). Briefly, immediately after removing the spleen from the immunized mouse, the tissue was homogenized in 10 ml of a denaturing solution containing 4.0 M guanine isothiocyanate, 0.25 M sodium citrate at pH 7.0, and 0.1 M 2-mercaptoethanol using a glass homogenizer. One ml of sodium acetate at a concentration of 2 M at pH 4.0 was mixed with the homogenized spleen. One ml of saturated phenol was also mixed with the denaturing solution containing the homogenized spleen. Two ml of a chloroform:isoamyl alcohol (24:1 v/v) mixture was added to this homogenate. The homogenate was mixed vigorously for ten seconds and maintained on ice for 15 minutes. The homogenate was then transferred to a thick-walled 50 ml polypropylene centrifuge tube (Fisher Scientific Company, Pittsburgh, PA). The solution was centrifuged at 10,000 x g for 20 minutes at 4°C. The upper RNA-containing aqueous layer was transferred to a fresh 50 ml polypropylene centrifuge tube and mixed with an equal volume of isopropyl alcohol. This solution was maintained at -20°C for at least one hour to precipitate the RNA. The solution containing the precipitated RNA was centrifuged at 10,000 x g for twenty minutes at 4°C. The pelleted total cellular RNA was collected and dissolved in 3 ml of the denaturing solution described above. Three mls of isopropyl alcohol was added to the resuspended total cellular RNA and vigorously mixed. This solution was maintained at -20°C for at least 1 hour to precipitate the RNA. The solution containing the precipitated RNA was centrifuged at 10,000 x g for ten minutes at 4°C. The pelleted RNA was washed once with a solution containing 75% ethanol. The pelleted RNA was dried under vacuum for 15 minutes and then resuspended in dimethyl pyrocarbonate (DEPC) treated (DEPC-H₂O) H₂O.

Poly A⁺ RNA for use in first strand cDNA synthesis was prepared from the above isolated total RNA using a spin-column kit (Pharmacia, Piscataway, NJ) as recommended by the manufacturer. The basic methodology has been described by Aviv and Leder in Proc. Natl. Acad. Sci., USA, 69:1408-1412 (1972). Briefly, one half of the total RNA isolated from a single immunized mouse spleen prepared as described above was resuspended in one ml of DEPC-treated dH₂O and maintained at 65°C for five minutes. One ml of 2x high salt loading buffer (100 mM Tris-HCL at pH 7.5, 1 M sodium chloride, 2.0 mM disodium ethylene diamine tetraacetic acid (EDTA) at pH 8.0, and 0.2% sodium dodecyl sulfate (SDS)) was added to the resuspended RNA and the mixture was allowed to cool to room temperature. The mixture was then applied to an oligo-dT (Collaborative Research Type 2 or Type 3 Bedford, MA) column that was previously prepared by washing the oligo-dT with a solution containing 0.1 M sodium hydroxide and 5 mM EDTA and then equilibrating the column with DEPC-treated dH₂O. The eluate was collected in a sterile polypropylene tube and reapplied to the same column after heating the eluate for 5 minutes at 65°C. The oligo dT column was then washed with 2 ml of high salt loading buffer consisting of 50 mM Tris-HCL at pH 7.5, 500 mM sodium chloride, 1 mM EDTA at pH 8.0 and 0.1% SDS. The oligo dT column was then washed with 2 ml of 1 X medium salt buffer (50 mM Tris-HCL at pH 7.5, 100 mM sodium chloride, 1 mM EDTA at pH 8.0 and 0.1% SDS). The mRNA was eluted with 1 ml of buffer consisting of 10 mM Tris-HCL at pH 7.5, 1 mM EDTA at pH 8.0 and 0.05% SDS. The messenger RNA was purified by extracting this solution with phenol/chloroform followed by a single extraction with 100% chloroform, ethanol precipitated and resuspended in DEPC treated dH₂O.

In preparation for PCR amplification, mRNA was used as a template for cDNA synthesis. In a typical 250 *µ*l reverse transcription reaction mixture, 5-10 *µ*g of spleen mRNA in water was first annealed with 500 ng (0.5 pmol) of either the 3' V_{H} primer (primer 12, Table I) or the 3' V_{L} primer (primer 9, Table II) at 65°C for 5 minutes. Subsequently, the mixture was adjusted to contain 0.8 mM dATP, 0.8 mM dCTP, 0.8 mM dGTP, 0.8 mM dTTP, 100 mM Tris-HCL (pH 8.6), 10 mM MgCl₂, 40 mM KCl, and 20 mM 2-ME. Moloney-Murine Leukemia Virus (Bethesda Research Laboratories (BRL), Gaithersburg, MD) Reverse transcriptase, 26 units, was added and the solution was incubated for 1 hour at 40°C. The resultant first strand cDNA was phenol extracted, ethanol precipitated and then used in the polymerase chain reaction (PCR) procedures described below for amplification of heavy and light chain sequences.

Primers used for amplification of heavy chain Fd fragments for construction of the M13IX30 library is shown in Table I. Amplification was performed in eight separate reactions, as described by Saiki et al. in Science, 239:487-491 (1988), each reaction containing one of the 5' primers (primers 2 to 9; SEQ ID NOS: 7 through 14, respectively) and one of the 3' primers (primer 12; SEQ ID NO: 17) listed in Table I. The remaining 5' primers, used for amplification in a single reaction, are either a degenerate primer (primer 1; SEQ ID NO: 6) or a primer that incorporates inosine at four degenerate positions (primer 10; SEQ ID NO: 15). The remaining 3' primer (primer 11; SEQ ID NO: 16) was used to construct Fv fragments. The underlined portion of the 5' primers incorporates an Xho I site and that of the 3' primer an Spe I restriction site for cloning the amplified fragments into the M13IX30 vector in a predetermined reading frame for expression.

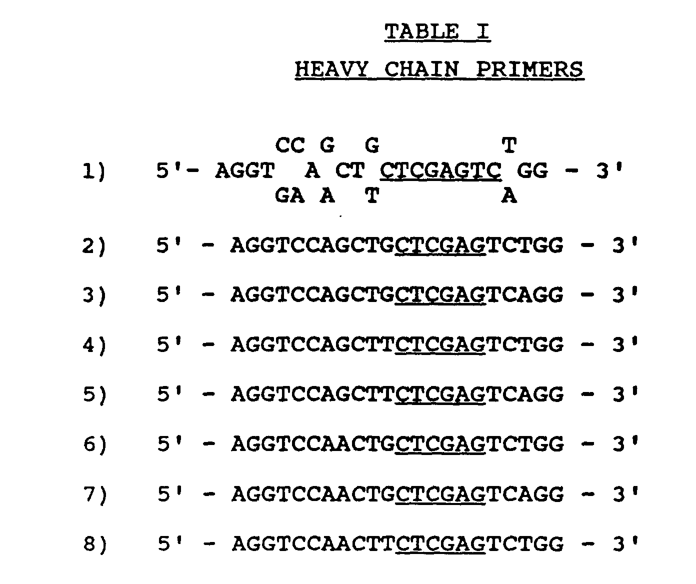

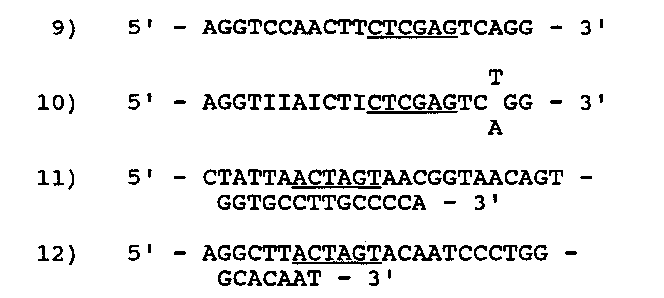

Primers used for amplification of mouse kappa light chain sequences for construction of the M13IX11 library are shown in Table II. These primers were chosen to contain restriction sites which were compatible with vector and not present in the conserved sequences of the mouse light chain mRNA. Amplification was performed as described above in five separate reactions, each containing one of the 5' primers (primers 3 to 7; SEQ ID NOS: 20 through 24, respectively) and one of the 3' primers (primer 9; SEQ ID NO: 26) listed in Table II. The remaining 3' primer (primer 8; SEQ ID NO: 25) was used to construct Fv fragments. The underlined portion of the 5' primers depicts a Sac I restriction site and that of the 3' primers an Xba I restriction site for cloning of the amplified fragments into the M13IX11 vector in a predetermined reading frame for expression.

**TABLE II**

| LIGHT CHAIN PRIMERS | |
|---|---|
| 1) | 5' - CCAGTTCCGAGCTCGTTGTGACTCAGGAATCT - 3' |
| 2) | 5' - CCAGTTCCGAGCTCGTGTTGACGCAGCCGCCC - 3' |
| 3) | 5' - CCAGTTCCGAGCTCGTGCTCACCCAGTCTCCA - 3' |
| 4) | 5' - CCAGTTCCGAGCTCCAGATGACCCAGTCTCCA - 3' |
| 5) | 5' - CCAGATGTGAGCTCGTGATGACCCAGACTCCA - 3' |
| 6) | 5' - CCAGATGTGAGCTCGTCATGACCCAGTCTCCA - 3' |
| 7) | 5' - CCAGTTCCGAGCTCGTGATGACACAGTCTCCA - 3' |
| 8) | 5' - GCAGCATTCTAGAGTTTCAGCTCCAGCTTGCC - 3' |
| 9) | 5' - GCGCCGTCTAGAATTAACACTCATTCCTGTTGAA - 3' |

PCR amplification for heavy and light chain fragments was performed in a 100 *µ*l reaction mixture containing the above described products of the reverse transcription reaction (≈5*µ* g of the cDNA-RNA hybrid), 300 nmol of 3' V_{H} primer (primer 12, Table I; SEQ ID NO: 17), and one of the 5' V_{H} primers (primers 2-9, Table I; SEQ ID NOS: 7 through 14, respectively) for heavy chain amplification, or, 300 nmol of 3' V_{L} primer (primer 9, Table II; SEQ ID NO: 26), and one of the 5' V_{L} primers (primers 3-7, Table II; SEQ ID NOS: 20 through 24, respectively) for each light chain amplification, a mixture of dNTPs at 200 mM, 50 mM KCl, 10 mM Tris-HCl (pH 8.3), 15 mM MgCl₂, 0.1% gelatin, and 2 units of Thermus aquaticus DNA polymerase. The reaction mixture was overlaid with mineral oil and subjected to 40 cycles of amplification. Each amplification cycle involved denaturation at 92°C for 1 minute, annealing at 52°C for 2 minutes, and elongation at 72°C for 1.5 minutes. The amplified samples were extracted twice with phenol/CHCl₃ and once with CHCl₃, ethanol-precipitated, and stored at -70°C in 10 mM Tris-HCl, pH 7.5 1 mM EDTA. The resultant products were used in constructing the M13IX30 and M13IX11 libraries (see below).

### Vector Construction

Two M13-based vectors, M13IX30 (SEQ ID NO: 1) and M13IX11 (SEQ ID NO: 2), were constructed for the cloning and propagation of Hc and Lc populations of antibody fragments, respectively. The vectors were constructed to facilitate the random joining and subsequent surface expression of antibody fragment populations.

M13IX30 (SEQ ID NO: 1), or the Hc vector, was constructed to harbor diverse populations of Hc antibody fragments. M13mp19 (Pharmacia, Piscataway, NJ) was the starting vector. This vector was modified to contain, in addition to the encoded wild type M13 gene VIII: (1) a pseudo-wild type gene VIII sequence with an amber stop codon between it and the restriction sites for cloning oligonucleotides; (2) Stu I restriction site for insertion of sequences by hybridization and, Spe I and Xho I restriction sites in-frame with the pseudo-wild type gene VIII for cloning Hc sequences; (3) sequences necessary for expression, such as a promoter, signal sequence and translation initiation signals; (4) two pairs of Hind III-Mlu I sites for random joining of Hc and Lc vector portions, and (5) various other mutations to remove redundant restriction sites and the amino terminal portion of Lac Z.

Construction of M13IX30 was performed in four steps. In the first step, an M13-based vector containing the pseudo gVIII and various other mutations was constructed, M13IX01F. The second step involved the construction of a small cloning site in a separate M13mp18 vector to yield M13IX03. This vector was then expanded to contain expression sequences and restriction sites for Hc sequences to form M13IX04B. The fourth and final step involved the incorporation of the newly constructed sequences in M13IX04B into M13IX01F to yield M13IX30.

Construction of M13IX01F first involved the generation of a pseudo wild-type gVIII sequence for surface expression of antibody fragments. The pseudo-wild type gene encodes the identical amino acid sequence as that of the wild type gene; however, the nucleotide sequence has been altered so that only 63% identity exists between this gene and the encoded wild type gene VIII. Modification of the gene VIII nucleotide sequence used for surface expression reduces the possibility of homologous recombination with the wild type gene VIII contained on the same vector. Additionally, the wild type M13 gene VIII was retained in the vector system to ensure that at least some functional, non-fusion coat protein would be produced. The inclusion of wild type gene VIII facilitates the growth of phage under conditions where there is surface expression of the polypeptides and therefore reduces the possibility of non-viable phage production from the fusion genes.

The pseudo-wild type gene VIII was constructed by chemically synthesizing a series of oligonucleotides which encode both strands of the gene. The oligonucleotides are presented in Table III.

**TABLE III**

| Pseudo-Wild Type Gene VIII Oligonucleotide Series | |
|---|---|
| Top Strand Oligonucleotides | Sequence (5' to 3') |
| VIII 03 | GATCC TAG GCT GAA GGC GAT GAC CCT GCT AAG GCT GC |
| VIII 04 | A TTC AAT AGT TTA CAG GCA AGT GCT ACT GAG TAC A |
| VIII 05 | TT GGC TAC GCT TGG GCT ATG GTA GTA GTT ATA GTT |
| VIII 06 | GGT GCT ACC ATA GGG ATT AAA TTA TTC AAA AAG TT |
| VIII 07 | T ACG AGC AAG GCT TCT TA |

| Bottom Strand Oligonucleotides | |
|---|---|
| VIII 08 | AGC TTA AGA AGC CTT GCT CGT AAA CTT TTT GAA TAA TTT |
| VIII 09 | AAT CCC TAT GGT AGC ACC AAC TAT AAC TAC TAC CAT |
| VIII 10 | AGC CCA AGC GTA GCC AAT GTA CTC AGT AGC ACT TG |
| VIII 11 | C CTG TAA ACT ATT GAA TGC AGC CTT AGC AGG GTC |
| VIII 12 | ATC GCC TTC AGC CTA G |

Except for the terminal oligonucleotides VIII 03 (SEQ ID NO: 27) and VIII 08 (SEQ ID NO: 32), the above oligonucleotides (oligonucleotides VIII 04-07 (SEQ ID NOS: 28 through 31, respectively) and VIII 09-12 (SEQ ID NOS: 33 through 36, respectively)) were mixed at 200 ng each in 10 *µ* l final volume, phosphorylated with T4 polynucleotide Kinase (Pharmacia) and 1 mM ATP at 37°C for 1 hour, heated to 70°C for 5 minutes, and annealed into double-stranded form by heating to 65°C for 3 minutes, followed by cooling to room temperature over a period of 30 minutes. The reactions were treated with 1.0 U of T4 DNA ligase (BRL) and 1 mM ATP at room temperature for 1 hour, followed by heating to 70°C for 5 minutes. Terminal oligonucleotides were then annealed to the ligated oligonucleotides. The annealed and ligated oligonucleotides yielded a double-stranded DNA flanked by a Bam HI site at its 5' end and by a Hind III site at its 3' end. A translational stop codon (amber) immediately follows the Bam HI site. The gene VIII sequence begins with the codon GAA (Glu) two codons 3' to the stop codon. The double-stranded insert was cloned in frame with the Eco RI and Sac I sites within the M13 polylinker. To do so, M13mp19 was digested with Bam HI (New England Biolabs, Beverley, MA) and Hind III (New England Biolabs) and combined at a molar ratio of 1:10 with the double-stranded insert. The ligations were performed at room temperature overnight in 1X ligase buffer (50 mM Tris-HCl, pH 7.8, 10 mM MgCl₂, 20 mM DTT, 1 mM ATP, 50 *µ*g/ml BSA) containing 1.0 U of T4 DNA ligase (New England Biolabs). The ligation mixture was transformed into a host and screened for positive clones using standard procedures in the art.

Several mutations were generated within the construct to yield functional M13IX01F. The mutations were generated using the method of Kunkel et al., Meth. Enzymol. 154:367-382 (1987), for site-directed mutagenesis. The reagents, strains and protocols were obtained from a Bio Rad Mutagenesis kit (Bio Rad, Richmond, CA) and mutagenesis was performed as recommended by the manufacturer.

Two Fok I sites were removed from the vector as well as the Hind III site at the end of the pseudo gene VIII sequence using the mutant oligonucleotides 5'-CATTTTTGCAGATGGCTTAGA-3' (SEQ ID NO: 37) and 5'-TAGCATTAACGTCCAATA-3' (SEQ ID NO: 38). New Hind III and Mlu I sites were also introduced at position 3919 and 3951 of M13IX01F. The oligonucleotides used for this mutagenesis had the sequences 5 '-ATATATTTTAGTAAGCTTCATCTTCT-3' (SEQ ID NO: 39) and 5'-GACAAAGAACGCGTGAAAACTTT-3' (SEQ ID NO: 40), respectively. The amino terminal portion of Lac Z was deleted by oligonucleotide-directed mutagenesis using the mutant oligonucleotide 5'-GCGGGCCTCTTCGCTATTGCTTAAGAAGCCTTGCT-3' (SEQ ID NO: 41). In constructing the above mutations, all changes made in a M13 coding region were performed such that the amino acid sequence remained unaltered. The resultant vector, M13IX01F, was used in the final step to construct M13IX30 (see below).

In the second step, M13mp18 was mutated to remove the 5' end of Lac Z up to the Lac i binding site and including the Lac Z ribosome binding site and start codon. Additionally, the polylinker was removed and a Mlu I site was introduced in the coding region of Lac Z. A single oligonucleotide was used for these mutagenesis and had the sequence 5'-AAACGACGGCCAGTGCCAAGTGACGCGTGTGAAATTGTTATCC-3' (SEQ ID NO: 42). Restriction enzyme sites for Hind III and Eco RI were introduced downstream of the Mlu I site using the oligonucleotide 5'-GGCGAAAGGGAATTCTGCAAGGCGATTAAGCTTGGG TAACGCC-3' (SEQ ID NO. 43). These modifications of M13mp18 yielded the precursor vector M13IX03.

The expression sequences and cloning sites were introduced into M13IX03 by chemically synthesizing a series of oligonucleotides which encode both strands of the desired sequence. The oligonucleotides are presented in Table IV.

**TABLE IV**

| M13IX30 Oligonucleotide Series | |
|---|---|
| Top Strand Oligonucleotides | Sequence (5' to 3') |
| 084 | GGCGTTACCCAAGCTTTGTACATGGAGAAAATAAAG |
| 027 | TGAAACAAAGCACTATTGCACTGGCACTCTTACCGT TACCGT |
| 028 | TACTGTTTACCCCTGTGACAAAAGCCGCCCAGGTCC AGCTGC |
| 029 | TCGAGTCAGGCCTATTGTGCCCAGGGATTGTACTAG TGGATCCG |

| Bottom Oligonucleotides | Sequence (5' to 3') |
|---|---|
| 085 | TGGCGAAAGGGAATTCGGATCCACTAGTACAATCCCTG |
| 031 | GGCACAATAGGCCTGACTCGAGCAGCTGGACCAGGGCG GCTT |
| 032 | TTGTCACAGGGGTAAACAGTAACGGTAACGGTAAGTGT GCCA |
| 033 | GTGCAATAGTGCTTTGTTTCACTTTATTTTCTCCATGT ACAA |

The above oligonucleotides of Table IV, except for the terminal oligonucleotides 084 (SEQ ID NO: 44) and 085 (SEQ ID NO: 48), were mixed, phosphorylated, annealed and ligated to form a double-stranded insert as described in Example I. However, instead of cloning directly into the intermediate vector the insert was first amplified by PCR. The terminal oligonucleotides were used as primers for PCR. Oligonucleotide 084 (SEQ ID NO: 44) contains a Hind III site, 10 nucleotides internal to its 5' end and oligonucleotide 085 (SEQ ID NO: 48) has an Eco RI site at its 5' end. Following amplification, the products were restricted with Hind III and Eco RI and ligated, as described in Example I, into the polylinker of M13mp18 digested with the same two enzymes. The resultant double stranded insert contained a ribosome binding site, a translation initiation codon followed by a leader sequence and three restriction enzyme sites for cloning random oligonucleotides (Xho I, Stu I, Spe I). The intermediate vector was named M13IX04.

During cloning of the double-stranded insert, it was found that one of the GCC codons in oligonucleotides 028 and its complement in 031 was deleted. Since this deletion did not affect function, the final construct is missing one of the two GCC codons. Additionally, oligonucleotide 032 (SEQ ID NO: 50) contained a GTG codon where a GAG codon was needed. Mutagenesis was performed using the oligonucleotide 5'-TAACGGTAAGAGTGCCAGTGC-3' (SEQ ID NO: 52) to convert the codon to the desired sequence. The resultant vector is named M13IX04B.

The third step in constructing M13IX30 involved inserting the expression and cloning sequences from M13IX04B upstream of the pseudo wild-type gVIII in M13IX01F. This was accomplished by digesting M13IX04B with Dra III and Bam HI and gel isolating the 700 base pair insert containing the sequences of interest. M13IX01F was likewise digested with Dra III and Bam HI. The insert was combined with the double digested vector at a molar ratio of 1:1 and ligated as described in Example I. The sequence of the final construct M13IX30, is shown in Figure 2 (SEQ ID NO: 1). Figure 1A also shows M13IX30 where each of the elements necessary for surface expression of Hc fragments is marked. It should be noted during modification of the vectors, certain sequences differed from the published sequence of M13mp18. The new sequences are incorporated into the sequences recorded herein.

M13IX11 (SEQ ID NO: 2), or the Lc vector, was constructed to harbor diverse populations of Lc antibody fragments. This vector was also constructed from M13mp19 and contains: (1) sequences necessary for expression, such as a promoter, signal sequence and translation initiation signals; (2) Eco RV restriction site for insertion of sequences by hybridization and Sac I and Xba I restriction sites for cloning of Lc sequences; (3) two pairs of Hind III-Mlu I sites for random joining of Hc and Lc vector portions, and (4) various other mutation to remove redundant restriction sites.

The expression, translation initiation signals, cloning sites, and one of the Mlu I sites were constructed by annealing of overlapping oligonucleotides as described above to produce a double-stranded insert containing a 5' Eco RI site and a 3' Hind III site. The overlapping oligonucleotides are shown in Table V and were ligated as a double-stranded insert between the Eco RI and Hind III sites of M13mp18 as described for the expression sequences inserted into M13IX03. The ribosome binding site (AGGAGAC) is located in oligonucleotide 015 and the translation initiation codon (ATG) is the first three nucleotides of oligonucleotide 016 (SEQ ID NO: 55).

**TABLE V**

| Oligonucleotide Series for Construction of Translation Signals in M13IX11 | |
|---|---|
| Oligonucleotide | Sequence (5' to 3') |
| 082 | CACC TTCATG AATTC GGC AAG GAGACA GTCAT |
| 015 | AATT C GCC AAG GAG ACA GTC AT |
| 016 | AATG AAA TAC CTA TTG CCT ACG GCA GCC GCT GGA TTG TT |
| 017 | ATTA CTC GCT GCC CAA CCA GCC ATG GCC GAG CTC GTG AT |
| 018 | GACC CAG ACT CCA GATATC CAA CAG GAA TGA GTG TTA AT |
| 019 | TCT AGA ACG CGT C |
| 083 | TTCAGGTTGAAGC TTA CGC GTT CTA GAA TTA ACA CTC ATT CCTGT |
| 021 | TG GAT ATC TGG AGT CTG GGT CAT CAC GAG CTC GGC CAT G |
| 022 | GC TGG TTG GGC AGC GAG TAA TAA CAA TCC AGC GGC TGC C |
| 023 | GT AGG CAA TAG GTA TTT CAT TAT GAC TGT CCT TGG CG |

Oligonucleotide 017 (SEQ ID NO: 56) contained a Sac I restriction site 67 nucleotides downstream from the ATG codon. The naturally occurring Eco RI site was removed and new Eco RI and Hind III sites were introduced downstream from the Sac I. Oligonucleotides 5'-TGACTGTCTCCTTGGCGTGTGAAATTGTTA-3' (SEQ ID NO: 63) and 5'-TAACACTCATTCCGGATGGAATTCTGGAGTCTGGGT-3' (SEQ ID NO: 64) were used to generate each of the mutations, respectively. The Lac Z ribosome binding site was removed when the original Eco RI site in M13mp19 was mutated. Additionally, when the new Eco RI and Hind III sites were generated, a spontaneous 100 bp deletion was found just 3' to these sites. Since the deletion does not affect the function, it was retained in the final vector.

In addition to the above mutations, a variety of other modifications were made to incorporate or remove certain sequences. The Hind III site used to ligate the double-stranded insert was removed with the oligonucleotide 5'-GCCAGTGCCAAGTGACGCGTTCTA-3' (SEQ ID NO: 65). Second Hind III and Mlu I sites were introduced at positions 3922 and 3952, respectively, using the oligonucleotides 5'-ATATATTTTAGTAAGCTTCATCTTCT-3' (SEQ ID NO: 66) for the Hind III mutagenesis and 5'-GACAAAGAACGCGTGAAAACTTT-3' (SEQ ID NO: 67) for the Mlu I mutagenesis. Again, mutations within the coding region did not alter the amino acid sequence.

The sequence of the resultant vector, M13IX11, is shown in Figure 3 (SEQ ID NO: 2). Figure 1B also shows M13IX11 where each of the elements necessary for producing a surface expression library between Lc fragments is marked.

### Library Construction

Each population of Hc and Lc sequences synthesized by PCR above are separately cloned into M13IX30 and M13IX11, respectively, to create Hc and Lc libraries.

The Hc and Lc products (5 *µ*g) are mixed, ethanol precipitated and resuspended in 20 *µ*l of NaOAc buffer (33 mM Tris acetate, pH 7.9, 10 mM Mg-acetate, 66 mM K-acetate, 0.5 mM DTT). Five units of T4 DNA polymerase is added and the reactions incubated at 30°C for 5 minutes to remove 3' termini by exonuclease digestion. Reactions are stopped by heating at 70°C for 5 minutes. M13IX30 is digested with Stu I and M13IX11 is digested with Eco RV. Both vectors are treated with T4 DNA polymerase as described above and combined with the appropriate PCR products at a 1:1 molar ratio at 10 ng/*µ*l to anneal in the above buffer at room temperature overnight. DNA from each annealing is electroporated into MK30-3 (Boehringer, Indianapolis, IN), as described below, to generate the Hc and Lc libraries.

E. coli MK30-3 is electroporated as described by Smith et al., Focus 12:38-40 (1990) which is incorporated herein by reference. The cells are prepared by inoculating a fresh colony of MK30-3 into 5 mls of SOB without magnesium (20 g bacto-tryptone, 5 g bacto-yeast extract, 0.584 g NaCl, 0.186 g KCl, dH₂O to 1,000 mls) and grown with vigorous aeration overnight at 37°C. SOB without magnesium (500 ml) is inoculated at 1:1000 with the overnight culture and grown with vigorous aeration at 37°C until the OD₅₅₀ is 0.8 (about 2 to 3 h). The cells are harvested by centrifugation at 5,000 rpm (2,600 x g) in a GS3 rotor (Sorvall, Newtown, CT) at 4°C for 10 minutes, resuspended in 500 ml of ice-cold 10% (v/v) sterile glycerol, centrifuged and resuspended a second time in the same manner. After a third centrifugation, the cells are resuspended in 10% sterile glycerol at a final volume of about 2 ml, such that the OD₅₅₀ of the suspension was 200 to 300. Usually, resuspension is achieved in the 10% glycerol that remained in the bottle after pouring off the supernate. Cells are frozen in 40 *µ*l aliquots in microcentrifuge tubes using a dry ice-ethanol bath and stored frozen at -70°C.

Frozen cells are electroporated by thawing slowly on ice before use and mixing with about 10 pg to 500 ng of vector per 40 *µ*l of cell suspension. A 40 *µ*l aliquot is placed in an 0.1 cm electroporation chamber (Bio-Rad, Richmond, CA) and pulsed once at 0°C using 4 kΩ parallel resistor 25 *µ*F, 1.88 KV, which gives a pulse length (τ) of ^{~}4 ms. A 10 *µ*l aliquot of the pulsed cells are diluted into 1 ml SOC (98 mls SOB plus 1 ml of 2 M MgCl₂ and 1 ml of 2 M glucose) in a 12- x 75-mm culture tube, and the culture is shaken at 37°C for 1 hour prior to culturing in selective media, (see below).

Each of the libraries are cultured using methods known to one skilled in the art. Such methods can be found in Sanbrook et al., Molecular Cloning: A Laboratory Manuel, Cold Spring Harbor Laboratory, Cold Spring Harbor, 1989, and in Ausubel et al., Current Protocols in Molecular Biology, John Wiley and Sons, New York, 1989, both of which are incorporated herein by reference. Briefly, the above 1 ml library cultures are grown up by diluting 50-fold into 2XYT media (16 g tryptone, 10 g yeast extract, 5 g NaCl) and culturing at 37°C for 5-8 hours. The bacteria are pelleted by centrifugation at 10,000 x g. The supernatant containing phage is transferred to a sterile tube and stored at 4°C.

Double strand vector DNA containing Hc and Lc antibody fragments are isolated from the cell pellet of each library. Briefly, the pellet is washed in TE (10 mM Tris, pH 8.0, 1 mM EDTA) and recollected by centrifugation at 7,000 rpm for 5' in a Sorval centrifuge (Newtown, CT). Pellets are resuspended in 6 mis of 10% Sucrose, 50 mM Tris, pH 8.0. 3.0 ml of 10 mg/*µ*l lysozyne is added and incubated on ice for 20 minutes. 12 mls of 0.2 M NaOH, 1% SDS is added followed by 10 minutes on ice. The suspensions are then incubated on ice for 20 minutes after addition of 7.5 mls of 3 M NaOAc, pH 4.6. The samples are centrifuged at 15,000 rpm for 15 minutes at 4°C, RNased and extracted with phenol/chloroform, followed by ethanol precipitation. The pellets are resuspended, weighed and an equal weight of CsCl₂ is dissolved into each tube until a density of 1.60 g/ml is achieved. EtBr is added to 600 *µ*g/ml and the double-stranded DNA is isolated by equilibrium centrifugation in a TV-1665 rotor (Sorval) at 50,000 rpm for 6 hours. These DNAs from each right and left half sublibrary are used to generate forty libraries in which the right and left halves of the randomized oligonucleotides have been randomly joined together.

The surface expression library is formed by the random joining of the Hc containing portion of M13IX30 with the Lc containing portion of M13IX11. The DNAs isolated from each library was digested separately with an excess amount of restriction enzyme. The Lc population (5 *µ*g) is digested with Hind III. The Hc (5 *µ*g) population is digested with Mlu I. The reactions are stopped by phenol/chloroform extraction followed by ethanol precipitation. The pellets are washed in 70% ethanol and resuspended in 20 *µ*l of NaOAc buffer. Five units of T4 DNA polymerase (Pharmacia) is added and the reactions incubated at 30°C for 5 minutes. Reactions are stopped by heating at 70°C for 5 minutes. The Hc and Lc DNAs are mixed to a final concentration of 10 ng each vector/*µ*l and allowed to anneal at room temperature overnight. The mixture is electroporated into MK30-3 cells as described above.

### Screening of Surface Expression Libraries

Purified phage are prepared from 50 ml liquid cultures of XL1 Blue™ cells (Stratagene, La Jolla, CA) which had been infected at a m.o.i. of 10 from the phage stocks stored at 4°C. The cultures are induced with 2 mM IPTG. Supernatants are cleared by two centrifugations, and the phage are precipitated by adding 1/7.5 volumes of PEG solution (25% PEG-8000, 2.5 M NaCl), followed by incubation at 4°C overnight. The precipitate is recovered by centrifugation for 90 minutes at 10,000 x g. Phage pellets are resuspended in 25 ml of 0.01 M Tris-HCl, pH 7.6, 1.0 mM EDTA, and 0.1% Sarkosyl and then shaken slowly at room temperature for 30 minutes. The solutions are adjusted to 0.5 M NaCl and to a final concentration of 5% polyethylene glycol. After 2 hours at 4°C, the precipitates containing the phage are recovered by centrifugation for 1 hour at 15,000 X g. The precipitates are resuspended in 10 ml of NET buffer (0.1 M NaCl, 1.0 mM EDTA, and 0.01 M Tris-HCl, pH 7.6), mixed well, and the phage repelleted by centrifugation at 170,000 X g for 3 hours. The phage pellets are resuspended overnight in 2 ml of NET buffer and subjected to cesium chloride centrifugation for 18 hours at 110,000 X g (3.86 g of cesium chloride in 10 ml of buffer). Phage bands are collected, diluted 7-hold with NET buffer, recentrifuged at 170,000 X g for 3 hours, resuspended, and stored at 4°C in 0.3 ml of NET buffer containing 0.1 mM sodium azide.

The BDP used for panning on streptavidin coated dishes is first biotinylated and then absorbed against UV-inactivated blocking phage (see below). The biotinylating reagents are dissolved in dimethylformamide at a ratio of 2.4 mg solid NHS-SS-Biotin (sulfosuccinimidyl 2-(biotinamido) ethyl-1,3'-dithiopropionate; Pierce, Rockford, IL) to 1 ml solvent and used as recommended by the manufacturer. Small-scale reactions are accomplished by mixing 1 *µ*l dissolved reagent with 43 *µ*l of 1 mg/ml BDP diluted in sterile bicarbonate buffer (0.1 M NaHCO₃, pH 8.6). After 2 hours at 25°C, residual biotinylating reagent is reacted with 500 *µ*l 1 M ethanolamine (pH adjusted to 9 with HCl) for an additional 2 hours. The entire sample is diluted with 1 ml TBS containing 1 mg/ml BSA, concentrated to about 50 *µ*l on a Centricon 30 ultrafilter (Amicon), and washed on the same filter three times with 2 ml TBS and once with 1 ml TBS containing 0.02% NaN₃ and 7 x 10¹² UV-inactivated blocking phage (see below); the final retentate (60-80 *µ* l) is stored at 4 °C. BDP biotinylated with the NHS-SS-Biotin reagent is linked to biotin via a disulfide-containing chain.

UV-irradiated M13 phage are used for blocking any biotinylated BDP which fortuitously binds filamentous phage in general. M13mp8 (Messing and Vieira, Gene 19: 262-276 (1982) ) is chosen because it carries two amber mutations, which ensure that the few phage surviving irradiation will not grow in the sup O strains used to titer the surface expression library. A 5 ml sample containing 5 x 10¹³ M13mp8 phage, purified as described above, is placed in a small petri plate and irradiated with a germicidal lamp at a distance of two feet for 7 minutes (flux 150 *µ*W/cm²). NaN₃ is added to 0.02% and phage particles concentrated to 10¹⁴ particles/ml on a Centricon 30-kDa ultrafilter (Amicon).

For panning, polystyrene petri plates (60 x 15 mm) are incubated with 1 ml of 1 mg/ml of streptavidin (BRL) in 0.1 M NaHCO₃ pH 8.6-0.02% NaN₃ in a small, air-tight plastic box overnight in a cold room. The next day streptavidin is removed and replaced with at least 10 ml blocking solution (29 mg/ml of BSA; 3 *µ*g/ml of streptavidin; 0.1 M NaHCO₃ pH 8.6-0.02% NaN₃) and incubated at least 1 hour at room temperature. The blocking solution is removed and plates are washed rapidly three times with Tris buffered saline containing 0.5% Tween 20 (TBS-0.5% Tween 20).

Selection of phage expressing antibody fragments which bind BDP is performed with 5 *µ*l (2.7 *µ*g BDP) of blocked biotinylated BDP reacted with a 50 *µ*l portion of the library. Each mixture is incubated overnight at 4°C, diluted with 1 ml TBS-0.5% Tween 20, and transferred to a streptavidin-coated petri plate prepared as described above. After rocking 10 minutes at room temperature, unbound phage are removed and plates washed ten times with TBS-0.5% Tween 20 over a period of 30-90 minutes. Bound phage are eluted from plates with 800 *µ*l sterile elution buffer (1 mg/ml BSA, 0.1 M HCl, pH adjusted to 2.2 with glycerol) for 15 minutes and eluates neutralized with 48 *µ*l 2 M Tris (pH unadjusted). A 20 *µ*l portion of each eluate is titered on MK30-3 concentrated cells with dilutions of input phage.

A second round of panning is performed by treating 750 *µ*l of first eluate from the library with 5 mM DTT for 10 minutes to break disulfide bonds linking biotin groups to residual biotinylated binding proteins. The treated eluate is concentrated on a Centricon 30 ultrafilter (Amicon), washed three times with TBS-0.5% Tween 20, and concentrated to a final volume of about 50 *µ*l. Final retentate is transferred to a tube containing 5.0 *µ*l (2.7 *µ*g BDP) blocked biotinylated BDP and incubated overnight. The solution is diluted with 1 ml TBS-0.5% Tween 20, panned, and eluted as described above on fresh streptavidin-coated petri plates. The entire second eluate (800 *µ* l) is neutralized with 48 *µ*l 2 M Tris, and 20 *µ*l is titered simultaneously with the first eluate and dilutions of the input phage. If necessary, further rounds of panning can be performed to obtain homogeneous populations of phage. Additionally, phage can be plaque purified if reagents are available for detection.

### Template Preparation and Sequencing

Templates are prepared for sequencing by inoculating a 1 ml culture of 2XYT containing a 1:100 dilution of an overnight culture of XL1 with an individual plaque from the purified population. The plaques are picked using a sterile toothpick. The culture is incubated at 37°C for 5-6 hours with shaking and then transferred to a 1.5 ml microfuge tube. 200 *µ*l of PEG solution is added, followed by vortexing and placed on ice for 10 minutes. The phage precipitate is recovered by centrifugation in a microfuge at 12,000 x g for 5 minutes. The supernatant is discarded and the pellet is resuspended in 230 *µ*l of TE (10 mM Tris-HCl, pH 7.5, 1 mM EDTA) by gently pipeting with a yellow pipet tip. Phenol (200 *µ*l) is added, followed by a brief vortex and microfuged to separate the phases. The aqueous phase is transferred to a separate tube and extracted with 200 *µ*l of phenol/chloroform (1:1) as described above for the phenol extraction. A 0.1 volume of 3 M NaOAc is added, followed by addition of 2.5 volumes of ethanol and precipated at -20°C for 20 minutes. The precipated templates are recovered by centrifugation in a microfuge at 12,000 x g for 8 minutes. The pellet is washed in 70% ethanol, dried and resuspended in 25 *µ*l TE. Sequencing was performed using a Sequenase™ sequencing kit following the protocol supplied by the manufacturer (U.S. Biochemical, Cleveland, OH).

### EXAMPLE II

### Cloning of Heavy and Light Chain Sequences Without Restriction Enzyme Digestion

This example shows the simultaneous incorporation of antibody heavy and light chain fragment encoding sequences into a M13IXHL-type vector with the use of restriction endonucleases.

For the simultaneous incorporation of heavy and light chain encoding sequences into a single coexpression vector, a M13IXHL vector was produced that contained heavy and light chain encoding sequences for a mouse monoclonal antibody (DAN-18H4; Biosite, San Diego, CA). The inserted antibody fragment sequences are used as complementary sequences for the hybridization and incorporation of Hc and Lc sequences by site-directed mutagenesis. The genes encoding the heavy and light chain polypeptides were inserted into M13IX30 (SEQ ID NO: 1) and M13IX11 (SEQ ID NO: 2), respectively, and combined into a single surface expression vector as described in Example I. The resultant M13IXHL-type vector is termed M13IX50.

The combinations were performed under conditions that facilitate the formation of one Hc and one Lc vector half into a single circularized vector. Briefly, the overhangs generated between the pairs of restriction sites after restriction with Mlu I or Hind III and exonuclease digestion are unequal (i.e., 64 nucleotides compared to 32 nucleotides). These unequal lengths result in differential hybridization temperatures for specific annealing of the complementary ends from each vector. The specific hybridization of each end of each vector half was accomplished by first annealing at 65°C in a small volume (about 100 *µ* g/*µ*l) to form a dimer of one Hc vector half and one Lc vector half. The dimers were circularized by diluting the mixture (to about 20 *µ*g/*µ*l) and lowering the temperature to about 25-37°C to allow annealing. T4 ligase was present to covalently close the circular vectors.

M13IX50 was modified such that it did not produce a functional polypeptide for the DAN monoclonal antibody. To do this, about eight amino acids were changed within the variable region of each chain by mutagenesis. The Lc variable region was mutagenized using the oligonucl side 5'-CTGAACCTGTCTGGGACCACAGTTGATGCTATAGGATCAGATCTAGAATTCATT TAGAGACTGGCCTGGCTTCTGC-3' (SEQ ID NO: 68). The Hc sequence was mutagenized with the oligonucleotide 5'-T C G A C C G T T G G T A G G A A T A A T G C A A T T A A T G GAGTAGCTCTAAATTCAGAATTCATCTACACCCAGTGCATCCAGTAGCT-3' (SEQ ID NO: 69). An additional mutation was also introduced into M13IX50 to yield the final form of the vector. During construction of an intermediate to M13IX50 (M13IX04 described in Example I), a six nucleotide sequence was duplicated in oligonucleotide 027 and its complement 032. This sequence, 5'TTACCG-3' was deleted by mutagenesis using the oligonucleotide 5'-GGTAAACAGTAACGGTAAGAGTGCCAG-3' (SEQ ID NO: 70). The resultant vector was designated M13IX53.

M13IX53 can be produced as a single stranded form and contains all the functional elements of the previously described M13IXHL vector except that it does not express functional antibody heteromers. The single-stranded vector can be hybridized to populations of single-stranded Hc and Lc encoding sequences for their incorporation into the vector by mutagenesis. Populations of single-stranded Hc and Lc encoding sequences can be produced by one skilled in the art from the PCR products described in Example I or by other methods known to one skilled in the art using the primers and teachings described therein. The resultant vectors with Hc and Lc encoding sequences randomly incorporated are propagated and screened for desired binding specificities as described in Example I.

Other vectors similar to M13IX53 and the vectors it's derived from, M13IX11 and M13IX30, have also been produced for the incorporation of Hc and Lc encoding sequences without restriction. In contrast to M13IX53, these vectors contain human antibody sequences for the efficient hybridization and incorporation of populations of human Hc and Lc sequences. These vectors are briefly described below. The starting vectors were either the Hc vector (M13IX30) or the Lc vector (M13IX11) previously described.

M13IX32 was generated from M13IX30 by removing the six nucleotide redundant sequence 5'-TTACCG-3' described above and mutation of the leader sequence to increase secretion of the product. The oligonucleotide used to remove the redundant sequence is the same as that given above. The mutation in the leader sequence was generated using the oligonucleotide 5'GGGCTTTTGCCACAGGGGT-3'. This mutagenesis resulted in the A residue at position 6353 of M13IX30 being changed to a G residue.

A decapeptide tag for affinity purification of antibody fragments was incorporated in the proper reading frame at the carboxy-terminal end of the Hc expression site in M13IX32. The oligonucleotide used for this mutagenesis was 5'-CGCCTT CAGCCTAAGAAGCGTAGTCCGGAACGTCGTACGGGTAGGATCCA CTAG-3' (SEQ ID NO: 71). The resultant vector was designated M13IX33. Modifications to this or other vectors are envisioned which include various features known to one skilled in the art. For example, a peptidase cleavage site can be incorporated following the decapeptide tag which allows the antibody to be cleaved from the gene VIII portion of the fusion protein.

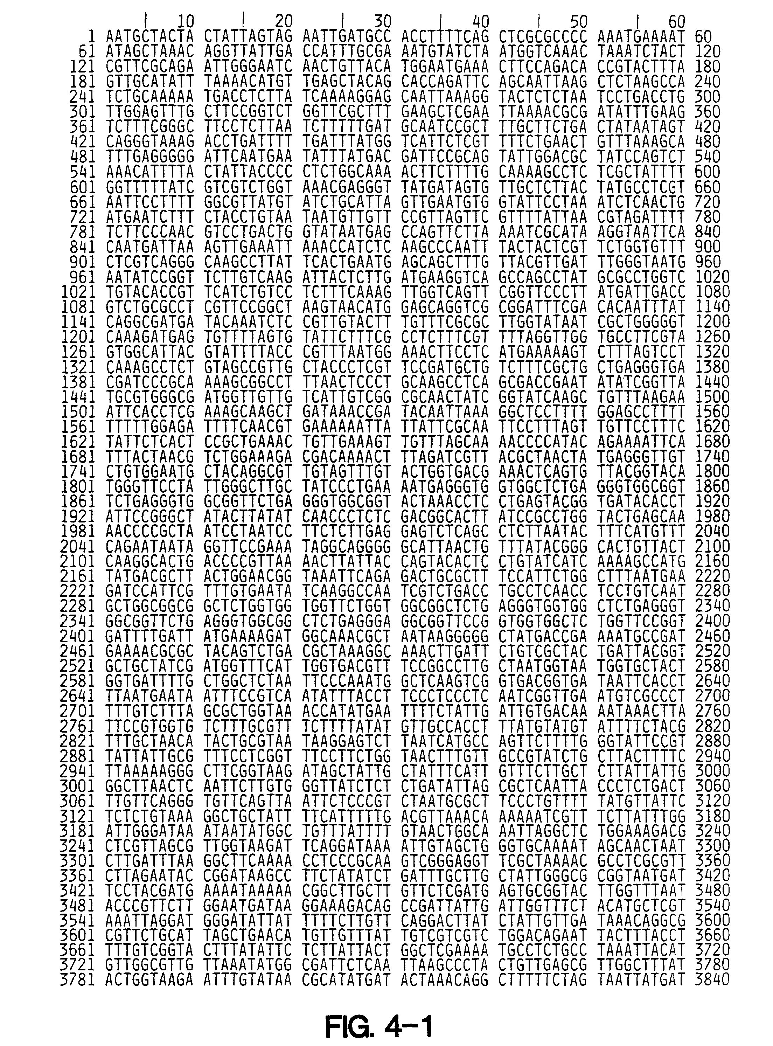

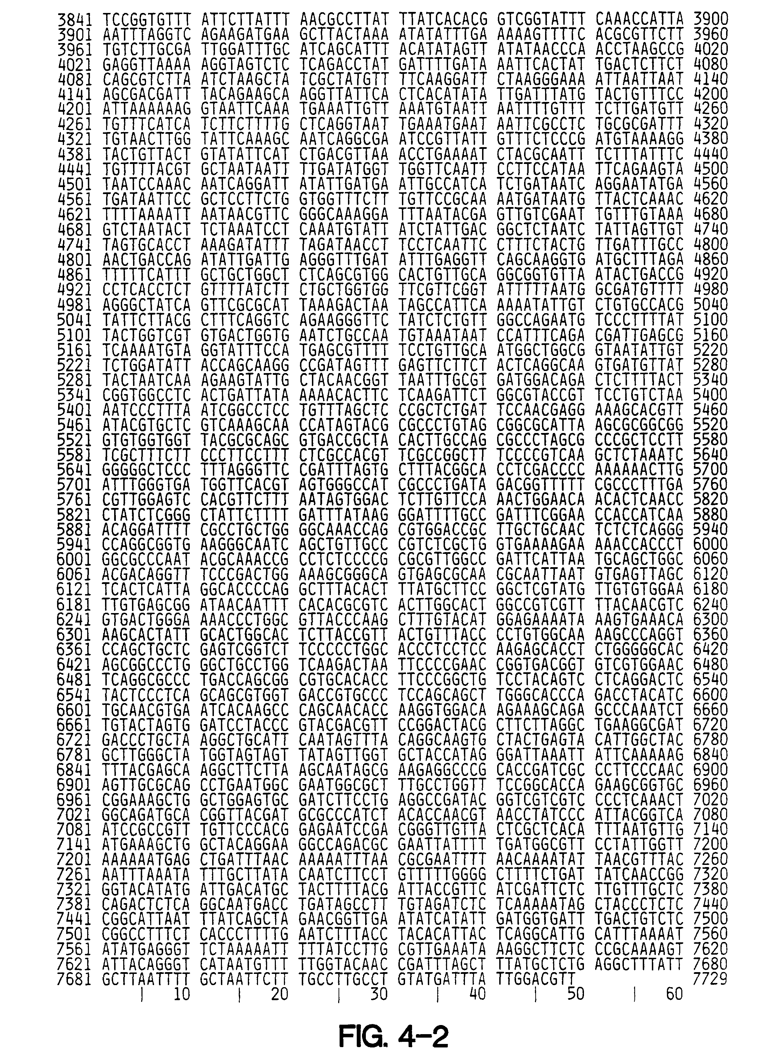

M13IX34 (SEQ ID NO: 3) was created from M13IX33 by cloning in the gene encoding a human IgG1 heavy chain. The reading frame of the variable region was changed and a stop codon was introduced to ensure that a functional polypeptide would not be produced. The oligonucleotide used for the mutagenesis of the variable region was 5'-CACCGGTTCGGGGAATTAGTCTTGACCAGGCAGCCCAGGGC-3' (SEQ ID NO: 72). The complete nucleotide sequence of this vector is shown in Figure 4 (SEQ ID NO: 3).

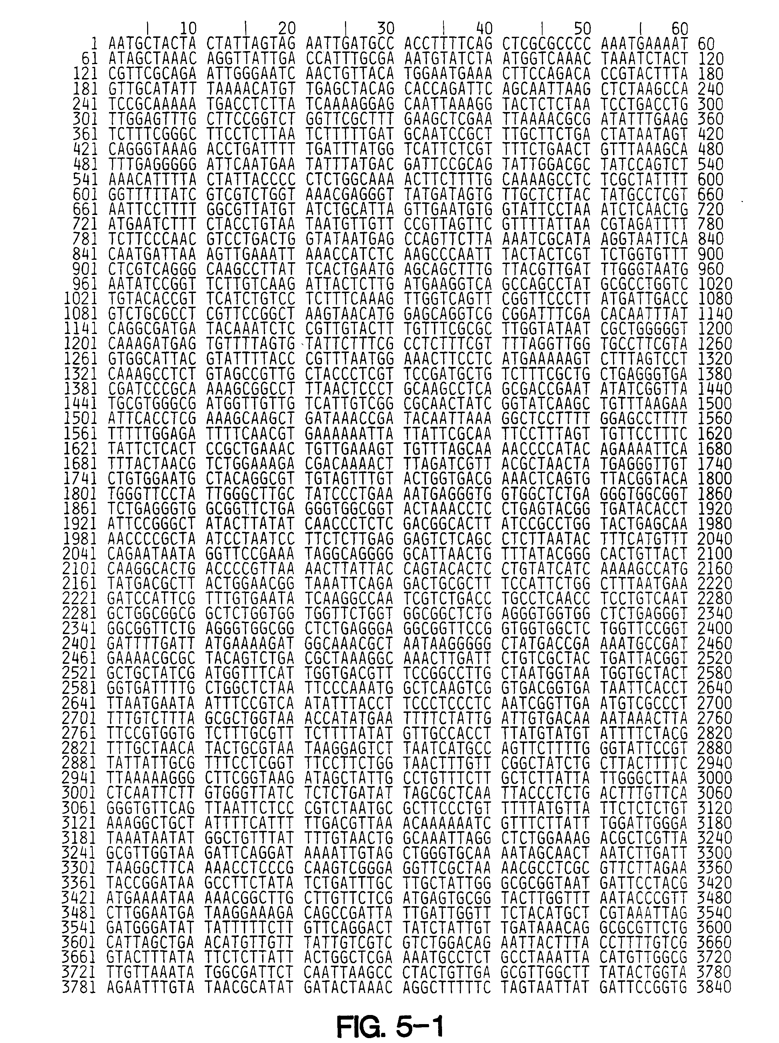

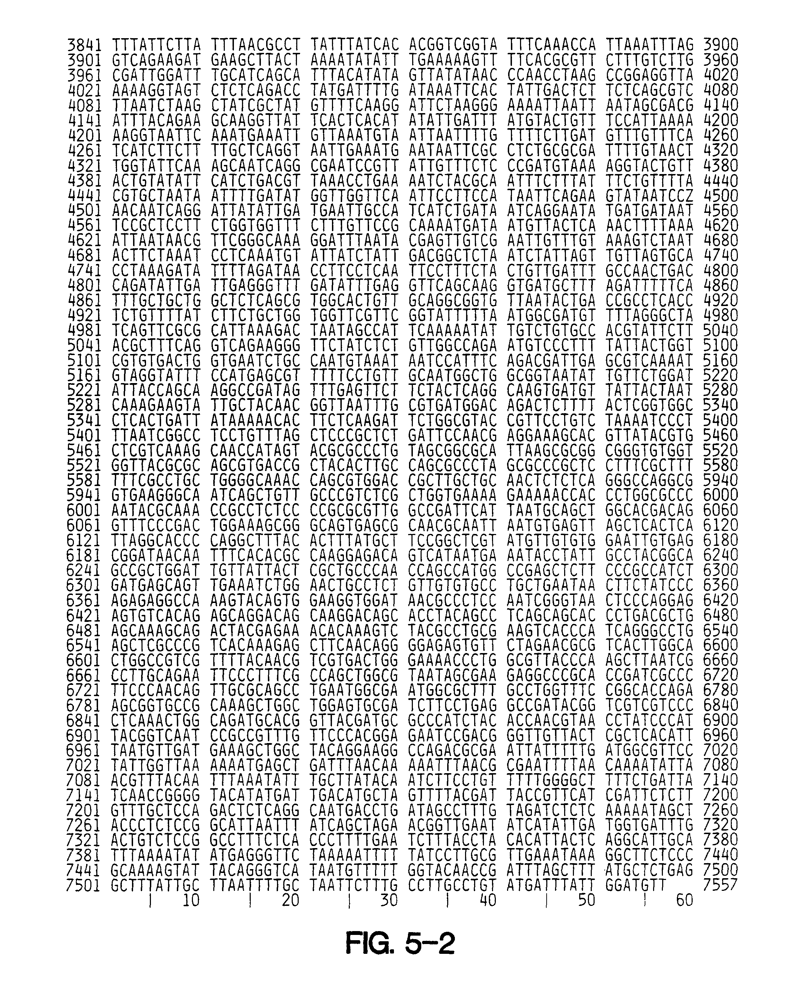

Several vectors of the M13IX11 series were also generated to contain similar modifications as that described for the vectors M13IX53 and M13IX34. The promoter region in M13IX11 was mutated to conform to the 35 consensus sequence to generate M13IX12. The oligonucleotide used for this mutagenesis was 5'-ATTCCACAC ATTATACGAGCCGGAAGCATAAAGTGTCAAGCCTGGGGTGCC-3' (SEQ ID NO: 73). A human kappa light chain sequence was cloned into M13IX12 and the variable region subsequently deleted to generate M13IX13 (SEQ ID NO: 4). The complete nucleotide sequence of this vector is shown in Figure 5 (SEQ ID NO: 4). A similar vector, designated M12X14, was also generated in which the human lambda light chain was inserted into M13IX12 followed by deletion of the variable region. The oligonucleotides used for the variable region deletion of M13IX13 and M13IX14 were 5'-CTG CTCATCAGATGGCGGGAAGAGCTCGGCCATGGCTGGTTG-3' (SEQ ID NO: 74) and 5'-GAACAGAGT GACCGAGGGGGCGAGCTCGGCCATGGCTGGTTG-3' (SEQ ID NO: 75), respectively.

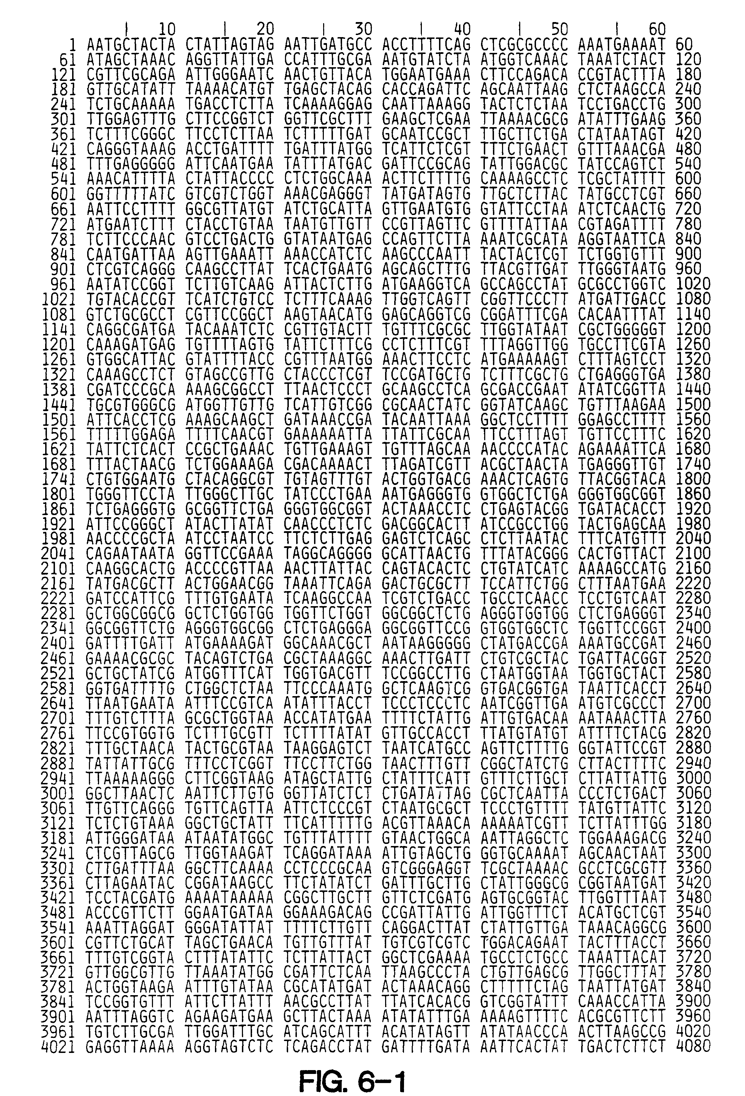

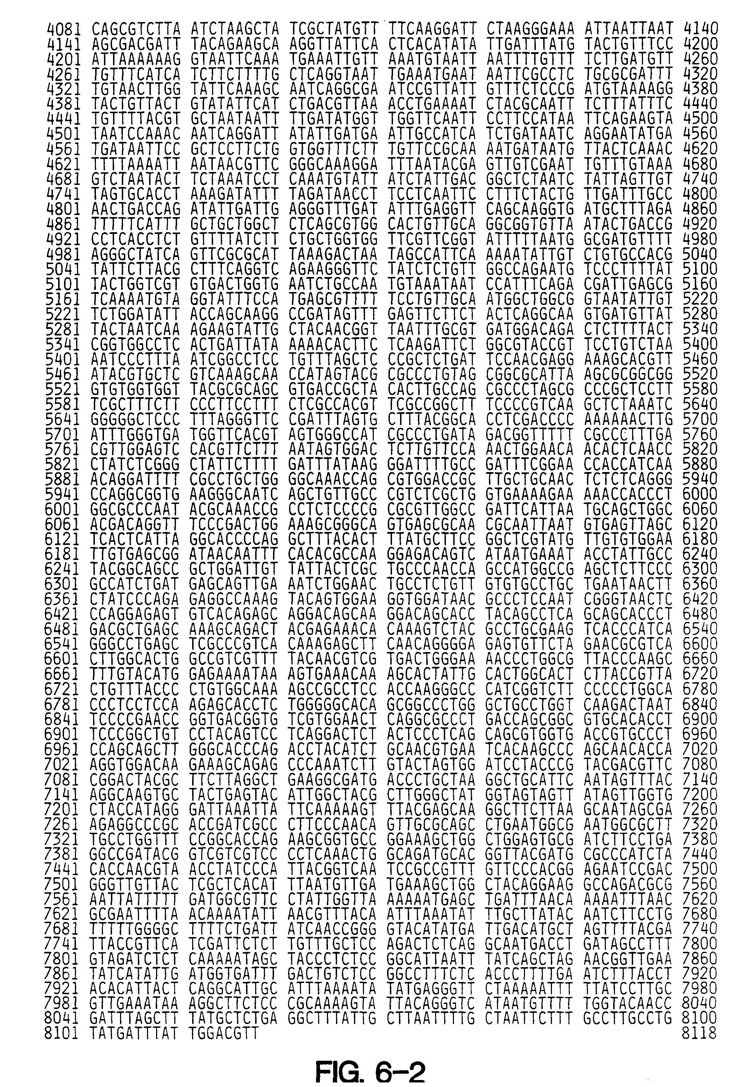

The Hc and Lc vectors or modified forms thereof can be combined using the methods described in Example I to produce a single vector similar to M13IX53 that allows the efficient incorporation of human Hc and Lc encoding sequences by mutagenesis. An example of such a vector is the combination of M13IX13 with M13IX34. The complete nucleotide sequence of this vector, M13IX60, is shown in Figure 6 (SEQ ID NO: 5).

Additional modifications to any of the previously described vectors can also be performed to generate vectors which allow the efficient incorporation and surface expression of Hc and Lc sequences. For example, to alleviate the use of uracil selection against wild-type template during mutagenesis procedures, the variable region locations within the vectors can be substituted by a set of palindromic restriction enzyme sites (i.e., two similar sites in opposite orientation). The palindromic sites will loop out and hybridize together during the mutagenesis and thus form a double-stranded substrate for restriction endonuclease digestion. Cleavage of the site results in the destruction of the wild-type template. The variable region of the inserted Hc or Lc sequences will not be affected since they will be in single stranded form.

Following the methods of Example I, single-stranded Hc or Lc populations can be produced by a variety of methods known to one skilled in the art. For example, the PCR primers described in Example I can be used in asymmetric PCR to generate such populations (Gelfand et al., "PCR Protocols: A Guide to Methods and Applications", Ed by M.A. Innis (1990)). Asymmetric PCR is a PCR method that differentially amplifies only a single strand of the double stranded template. Such differential amplification is accomplished by decreasing the primer amount for the undesirable strand about 10-fold compared to that for the desirable strand. Alternatively, single-stranded populations can be produced from double-stranded PCR products generated as described in Example I except that the primer(s) used to generate the undesirable strand of the double-stranded products is first phosphorylated at its 5' end with a kinase. The resultant products can then be treated with a 5' to 3' exonuclease, such as lambda exonuclease (BRL, Bethesda, MD) to digest away the unwanted strand.

Single-stranded Hc and Lc populations generated by the methods described above or by others known to one skilled in the art are hybridized to complementary sequences encoded in the previously described vectors. The population of the sequences are subsequently incorporated into a double-stranded form of the vector by polymerase extension of the hybridized templates. Propagation and surface expression of the randomly combined Hc and Lc sequences are performed as described in Example I.

## Claims

1. A composition of matter comprising a plurality of expression vectors containing a plurality of first and second DNA sequences encoding first and second polypeptides which form a heteromeric receptor, one or both of said first and second DNA sequences being operatively linked to a gene encoding a surface protein of a cell.

2. A composition as claimed in claim 1, wherein said heteromeric receptor exhibits binding activity toward a preselected molecule.

3. A composition as claimed in claim 1 or claim 2, wherein said heteromeric receptor is selected from antibodies, T cell receptors, integrins, hormone receptors, transmitter receptors and functional heteromeric portions thereof.

4. A composition as claimed in claim 3, wherein said heteromeric receptor is selected from antibodies, T cell receptors and functional heteromeric portions thereof.

5. A composition as claimed in any one of claims 1 to 4, wherein said expression vectors are filamentous bacteriophage selected from M13, fd and fl.

6. A composition as claimed in claim 5, wherein said gene encoding a surface protein encodes a filamentous bacteriophage gene VIII coat protein.

7. A plurality of cells comprising a composition of any one of claims 1 to 6, one or both of said receptor polypeptides being expressed as fusion proteins on the surface of said cells.

8. A vector comprising sequences necessary for the coexpression of two or more inserted DNA sequences encoding polypeptides which form heteromeric receptors and two genes encoding a filamentous bacteriophage coat protein, one of said genes capable of being operationally linked to one of said two or more inserted DNA sequences, such that said DNA sequence can be expressed as a fusion protein on the surface of a filamentous bacteriophage or as a soluble polypeptide.

## Patentansprüche

1. Zusammensetzung aus einem Material, umfassend eine Vielzahl von Expressionsvektoren, enthaltend eine Vielzahl von ersten und zweiten DNA-Sequenzen, die für erste und zweite Polypeptide kodieren, welche einen heteromeren Rezeptor bilden, wobei eine oder beide dieser ersten und zweiten DNA-Sequenzen funktionsfähig an ein Gen geknüpft ist, das für ein Oberflächenprotein einer Zelle kodiert.

2. Zusammensetzung nach Anspruch 1, wobei der heteromere Rezeptor eine Bindungsaktivität gegenüber einem zuvor gewählten Molekül zeigt.

3. Zusammensetzung nach Anspruch 1 oder Anspruch 2, wobei der heteromere Rezeptor gewählt ist aus Antikörpern, T-Zell-Rezeptoren, Integrinen, Hormonrezeptoren, Transmitter-Rezeptoren und funktionellen heteromeren Abschnitten davon.

4. Zusammensetzung nach Anspruch 3, wobei der heteromere Rezeptor gewählt ist aus Antikörpern, T-Zell-Rezeptoren und funktionellen heteromeren Abschnitten davon.

5. Zusammensetzung nach einem der Ansprüche 1 bis 4, wobei die Expressionsvektoren filamentöser Bakteriophage sind, der gewählt ist aus M13, fd und f1.

6. Zusammensetzung nach Anspruch 5, wobei das für ein Oberflächenprotein kodierende Gen für ein filamentöses Bakteriophagen-Gen-VIII-Hüllprotein kodiert.

7. Vielzahl von Zellen, umfassend eine Zusammensetzung nach einem der Ansprüche 1 bis 6, wobei eines oder beide der Rezeptor-Polypeptide als Fusionsproteine auf der Oberfläche dieser Zellen exprimiert werden.

8. Vektor, umfassend Sequenzen, die für die Coexpression zweier oder mehrerer insertierter DNA-Sequenzen, die für Polypeptide kodieren, welche heteromere Rezeptoren bilden, erforderlich sind, und zwei Gene, die für ein filamentöses Bakteriophagen-Hüllprotein kodieren, wobei eines der Gene funktionsfähig an eine der zwei oder mehr insertierten DNA-Sequenzen knüpfbar ist, so dass die DNA-Sequenz als ein Fusionsprotein an der Oberfläche eines filamentösen Bakteriophagen oder als ein lösliches Polypeptid exprimierbar ist.

## Revendications

1. Composition de matière comprenant une pluralité de vecteurs d'expression contenant une pluralité de première et seconde séquences d'ADN codant un premier et un second polypeptides qui forment un récepteur hétéromère, l'une ou les deux dites première et seconde séquences d'ADN étant liées de façon opérationnelle à un gène codant une protéine de surface d'une cellule.

2. Composition selon la revendication 1, dans laquelle ledit récepteur hétéromère présente une activité de liaison vis-à-vis d'une molécule présélectionnée.

3. Composition selon la revendication 1, dans laquelle ledit récepteur hétéromère est choisi parmi des anticorps, des récepteurs de cellules T, des intégrines, des récepteurs d'hormone, des récepteurs de transmetteur et des parties hétéromères fonctionnelles de ceux-ci.

4. Composition selon la revendication 3, dans laquelle le récepteur hétéromère est sélectionné parmi des anticorps, des récepteurs de cellules T et des parties hétéromères fonctionnelles de ceux-ci.

5. Composition selon l'une quelconque des revendications 1 à 4, dans laquelle lesdits vecteurs d'expression sont des bactériophages filamenteux sélectionnés parmi M13, fd et fl.

6. Composition selon la revendication 5, dans laquelle ledit gène codant une protéine de surface code une protéine d'enveloppe du gène VIII de bactériophage filamenteux.

7. Une pluralité de cellules comprenant une composition selon l'une quelconque des revendications 1 à 6, l'un ou les deux dits polypeptides de récepteur étant exprimés sous forme de protéine de fusion sur la surface desdites cellules.

8. Vecteur comprenant des séquences nécessaires pour la coexpression de deux ou plusieurs séquences d'ADN insérées codant des polypeptides qui forment des récepteurs hétéromères et deux gènes codant une protéine d'enveloppe de bactériophage filamenteux, l'un desdits gènes étant apte à être lié de façon opérationnelle à l'une desdites deux ou plusieurs séquences d'ADN insérées, de telle sorte que ladite séquence d'ADN puisse être exprimée comme une protéine de fusion sur la surface d'un bactériophage filamenteux ou comme un polypeptide soluble.
